(19) Europäisches Patentamt
European Patent Office
Office européen des brevets

(11) **EP 3 685 798 B1**

(12) **EUROPÄISCHE PATENTSCHRIFT**

(45) Veröffentlichungstag und Bekanntmachung des Hinweises auf die Patenterteilung:
**23.02.2022 Patentblatt 2022/08**

(51) Internationale Patentklassifikation (IPC):
***A61C 13/00*** *(2006.01)*     ***A61C 13/08*** *(2006.01)*

(21) Anmeldenummer: **20157084.3**

(22) Anmeldetag: **28.02.2018**

(52) Gemeinsame Patentklassifikation (CPC):
**A61C 13/0022; A61C 13/08; A61K 6/836; A61K 6/853; A61K 6/887**

(54) **FRÄSROHLING ZUR HERSTELLUNG EINER INDIREKTEN DENTALEN RESTAURATION, ENTSPRECHENDE VERWENDUNGEN UND VERFAHREN**

GRINDING BLANK FOR PRODUCING AN INDIRECT DENTAL RESTORATION, CORRESPONDING USES AND METHOD

FRAISAGE ÉBAUCHE DESTINÉ À LA FABRICATION D'UNE RESTAURATION DENTAIRE INDIRECT, USAGES ET PROCÉDÉ CORRESPONDANT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.03.2017 DE 102017105841**

(43) Veröffentlichungstag der Anmeldung:
**29.07.2020 Patentblatt 2020/31**

(62) Dokumentnummer(n) der früheren Anmeldung(en) nach Art. 76 EPÜ:
**18159074.6 / 3 375 407**

(73) Patentinhaber: **VOCO GmbH**
**27472 Cuxhaven (DE)**

(72) Erfinder:
• **Maletz, Reinhard**
 **27472 Cuxhaven (DE)**
• **Fontein, Nils**
 **27474 Cuxhaven (DE)**
• **Oldenburger, Daniel**
 **27476 Cuxhaven (DE)**
• **Plaumann, Manfred Thomas**
 **27472 Cuxhaven (DE)**

(74) Vertreter: **Eisenführ Speiser**
**Patentanwälte Rechtsanwälte PartGmbB**
**Postfach 10 60 78**
**28060 Bremen (DE)**

(56) Entgegenhaltungen:
JP-A- H0 856 965     JP-A- H01 275 510
US-A1- 2014 200 284     US-A1- 2014 220 512
US-A1- 2016 136 059

**Beschreibung**

Die vorliegende Erfindung betrifft einen Fräsrohling zur Herstellung einer indirekten dentalen Restauration, ein Verfahren zur Herstellung eines solchen Fräsrohlings oder eines daraus gefertigten Formkörpers zur Verwendung als indirekte dentale Restauration, einen Kit zur Herstellung von indirekten dentalen Restaurationen sowie ein Verfahren zur indirekten dentalen Restauration. Die Erfindung wird in den Ansprüchen definiert.

Das Dokument US2014200284 A offenbart eine Zusammensetzung, die zur Herstellung von Fräsrohlingen verwendet werden kann.

Indirekte dentale Restaurationen (auch indirekte prothetische Versorgungen genannt) bilden einen Ersatz für Zähne und Teile von Zähnen und umfassen insbesondere Formkörper, die aus der Gruppe ausgewählt sind, die aus Inlay, Onlay, Teilkronen, Kronen, Veneer (Verblendung) und Brücke besteht. Die vorliegende Erfindung betrifft sowohl temporäre als auch permanente Versorgungen, d.h. sowohl temporäre als auch permanente indirekte dentale Restaurationen.

Der Begriff "Fräsrohling" (alternative, gleichrangige Bezeichnungen: "dentaler Fräsrohling", "dentaler Fräsblock" oder "Fräsblank") bezeichnet für die Zwecke des vorliegenden Textes sowohl Fräsrohlinge, die aus Kunststoff bestehen (und somit keinen Füllstoff umfassen), als auch solche Fräsrohlinge, die aus einem kunststoffbasierten Komposit (auch "dentales Komposit" genannt) bestehen. Unter einem "kunststoffbasierten Komposit (d.h. einem "dentalen Kompositmaterial") versteht der Fachmann eine härtbare oder eine gehärtete dentale Zusammensetzung, die wenigstens eine härtbare flüssige oder eine gehärtete feste Harzphase (Kunststoffphase) sowie eine darin dispergierte Feststoffphase umfasst, wobei die Feststoffphase Füllstoffe unterschiedlicher Art und Menge enthalten kann und wobei die härtbare dentale Zusammensetzung ein oder mehrere Polymerisationsinitiator(en) und die härtbare oder gehärtete dentale Zusammensetzung gegebenenfalls übliche Additive wie Inhibitoren, Farbmittel, Stabilisatoren, etc. enthalten kann.

Ein noch nicht gehärteter (härtbarer) Kunststoff oder ein noch nicht gehärtetes (härtbares) kunststoffbasiertes Komposit kann entweder chemisch und/oder thermisch und/oder photochemisch mittels Strahlung polymerisiert werden. Fräsrohlinge zur Herstellung einer indirekten dentalen Restauration, die aus Kunststoff oder einem kunststoffbasierten Komposit bestehen, sind aus dem Stand der Technik bekannt. Sie sind insbesondere zur Herstellung von Dentalprothesen in einem CAD/CAM-Verfahren geeignet und vorgesehen.

Der technische Fortschritt computergesteuerter Maschinen brachte es mit sich, dass Fräsmaschinen entwickelt wurden, die in der Lage sind, in kürzester Zeit und mit minimalem Aufwand prothetische Versorgungen (d.h. indirekte dentale Restaurationen) mit äußerst hoher Präzision herzustellen. Vor diesem Hintergrund entwickelte sich die sogenannte "Digitale Zahnmedizin". Sie ist heutzutage in der Zahnmedizintechnik von überragender und stetig weiter zunehmender Bedeutung.

Gefräst wurden anfangs ausschließlich keramische oder metallische Materialien, doch mit Entwicklung von immer besser der natürlichen Zahnhartsubstanz angepassten Kunststoffen und kunststoffbasierten Kompositen wurden auch entsprechende Substanzen für die Verwendung als Fräsrohling interessant.

Im Zuge der Digitalisierung in der modernen Zahnheilkunde nehmen somit indirekte CAD/CAM gefertigte Restaurationen auf keramischer und auf Kunststoffbasis mehr und mehr an Bedeutung zu. Während sich keramikbasierte Restaurationen bereits klinisch bewährt haben, vgl. die nachfolgend angeführten Dokumente [1-7], nimmt in jüngerer Zeit auch die Bedeutung kunststoffbasierter Komposite zu. Als keramische Materialien kommen unter anderem Feldspatkeramik, Leucitkeramik und Lithiumdisilikatkeramik zum Einsatz; als nachteilig an derartigen keramischen Materialien hat sich jedoch deren Sprödigkeit erwiesen, so dass Frakturen und Chippings auftreten, vgl. die Dokumente [3-4,7-9]. Kunststoffe und kunststoffbasierte Komposite, insbesondere kunststoffbasierte Komposite für den Einsatz in CAD/CAM-Verfahren, weisen im Vergleich mit keramischen Materialien aufgrund ihrer höheren Elastizität Vorteile auf. Vorteile im Vergleich mit keramischen Materialien bieten kunststoffbasierte Materialien (reiner Kunststoff oder kunststoffbasierte Komposite) auch hinsichtlich ihrer leichteren Reparierbarkeit.

Erste vergleichende in vitro-Studien von CAD/CAM-gefertigten Kronen aus Keramik und kunststoffbasiertem Material (reiner Kunststoff bzw. kunststoffbasiertes Komposit) zeigen, dass mit beiden Produktklassen vergleichbare mechanische Eigenschaften erreicht werden können, vgl. hierzu die Dokumente [8 bis 12]. Bis zum Jahre 2010 gab es allerdings kaum Daten zur Retention von CAD/CAM-gefertigten kunststoffbasierten Kronen (aus Kunststoff oder einem kunststoffbasierten Komposit) oder sonstigen kunststoffbasierten indirekten dentalen Restaurationen. In einem entsprechenden Review (vgl. Dokument [13] von 2010) heißt es: " No study has investigated into composite or fiberreinforced composite crowns". Mit der steigenden Bedeutung von kunststoffbasierten CAD/CAM Materialien haben sich inzwischen mehrere universitäre Arbeitsgruppen mit dem Einfluss unterschiedlicher Parameter auf die Retention von CAD/CAM gefertigten Restaurationen beschäftigt, vgl. die Dokumente [17 bis 21]. So wurden unter anderem der Einfluss der Oberflächenvorbehandlung, des Befestigungsmaterials und des CAD/CAM Materials untersucht. Um die Retention zu bestimmen, wurden Scherhaftungs-, (Mikro-)Zughaftungs- sowie Kronenabzugsversuche durchgeführt, wobei letztere die klinische Situation am besten nachstellen.

In in vitro-Studien, die keramik- und kunststoffbasierte CAD/CAM-Materialien (Materialien zur Herstellung indirekter dentaler Restaurationen) vergleichen, wurde insgesamt eine bessere Retention der keramischen Materialien gefunden:

B. Stawarczyk untersuchte die Zugfestigkeit von adhäsiv befestigten kunststoffbasierten Kronen in Abhängigkeit vom Adhäsivsystem und vom Befestigungskomposit, vgl. Dokument [22]. Dabei wurde eine schlechtere Retention gefunden als für Keramikkronen, die mit dem gleichen Versuchsdesign untersucht wurden. "The investigated CAD/CAM nanocomposite combined with different pre-treatment methods and resin composite cements showed lower tensile bond strength tested with the same laboratory testing assay on dentin abutments compared to zirconia crowns [...] orglassceramic crowns".

T. Basler untersuchte die Zughaftung von adhäsiv befestigten CAD/CAM gefertigten kunststoffbasierten Kronen in Abhängigkeit von der Vorbehandlung und vom Befestigungsmaterial, vgl. Dokument [23]. Als Kontrollgruppe dienten dabei Glasskeramikkronen. Die Keramikkronen zeigten in den Untersuchungen signifikant höhere Haftwerte als die kunststoffbasierten Kronen: "The adhesively luted glass ceramic crowns [...] showed the highest tensile strength compared to all other test groups before and after mechanical thermocycling loading" und "In summary, the CAD/CAM resin crowns showed significantly lower tensile strength than the control group."

R. Frankenberger untersuchte den Einfluss von unterschiedlichen Vorbehandlungen und unterschiedlichen Besfestigungskompositen auf die Mikrozughaftung, vgl. Dokument [24]. Dabei wurden CAD/CAM Materialien aus Komposit, Lithiumdisilikatkeramik, zirkonverstärkter Lithiumsilikatkeramik und polymerinfiltrierter Keramik verglichen. Die höchsten Haftwerte wurden für die keramischen CAD/CAM Materialien gefunden.

Diese Ergebnisse werden durch erste klinische Studien belegt. Entsprechend schreibt R. Shetty (vgl. Dokument [25]) "Recent studies concluded that Resin Nano Ceramics tends to debond at the luting cement-crown interface especially used in cases of implant single crowns. Since resin matrix ceramics are resilient, elastic deformation occurs within the crown and this stress concentration may be transferred to the adhesive layer leading to debonding issues. The material is no longer considered an indication for crowns".

In einer 3-jährigen klinischen Studie beobachtete S. Vanoorbeek (vgl. Dokument [26]) bessere Überlebensraten von Keramikkronen (97,2% und 81,2%) im Vergleich zu kunststoffbasierten Kronen (87,9% und 55,6%).

In einer weiteren klinischen Studie zu kunststoffbasierten Kronen (vgl. Dokument [27]) wurden Kompositkronen (RNC) auf zwei unterschiedlichen Typen von Zirkonoxidabutments befestigt. Nach einem Jahr wurde eine Überlebensrate von nur 14% beobachtet. Das Versagen wurde auf die kunststoffbasierten Kronen zurückgeführt. "For this reason, we hypothesize that the clinical debonding of the RNC crowns in our study might have to do with the RNC material, rather than with the used cement". Als Folge wurde beschlossen, die verbliebenen kunststoffbasierten Kronen durch Keramikkronen zu ersetzen.

Es besteht daher ein beträchtlicher Bedarf an indirekten dentalen Restaurationen aus Kunststoff oder einem kunststoffbasierten Komposit, die sich durch eine besonders gute und im Vergleich mit dem Stand der Technik vorzugsweise verbesserte Retention auszeichnen. In gleicher Weise besteht ein hoher Bedarf an entsprechenden Fräsrohlingen zur Herstellung solcher indirekten dentalen Restaurationen.

Ein entsprechender Bedarf besteht hinsichtlich eines vorteilhaften Verfahrens zur Herstellung eines solchen Fräsrohlings oder eines daraus gefertigten Formkörpers zur Verwendung als indirekte dentale Restauration, wobei die Fertigung des Formkörpers aus dem Fräsrohling durch Fräsen, vorzugsweise mittels eines CAD/CAM-Verfahren erfolgen sollte.

Und überdies besteht ein entsprechender Bedarf an einem verbesserten Verfahren zur indirekten dentalen Restauration.

Es war eine primäre Aufgabe der vorliegenden Erfindung, Fräsrohlinge zur Herstellung einer indirekten dentalen Restauration mit verbesserter Retention anzugeben. Gemäß weiteren Aspekten der vorliegenden Erfindung war es eine weitere Aufgabe, entsprechende indirekte dentale Restaurationen anzugeben, entsprechende Verfahren zur Herstellung eines solchen Fräsrohlings anzugeben, eine entsprechende Verwendung eines solchen Fräsrohlings anzugeben sowie entsprechende Kits zur Herstellung von indirekten dentalen Restaurationen und Verfahren zur indirekten dentalen Restauration anzugeben, wobei immer das Ziel der verbesserten Retention anzustreben war.

Eine verbesserte Retention lässt sich in Laboruntersuchungen durch entsprechend gute Haftwerte nach Wasserlagerung in entsprechend konzipierten Tests abschätzen. Solche Tests sind weiter unten in den Beispielen angegeben. Es war eine spezifische Aufgabe der vorliegenden Erfindung, Fräsrohlinge etc. anzugeben, die in den genannten Tests besonders überzeugende Hafteigenschaften auch nach Wasserlagerung besitzen.

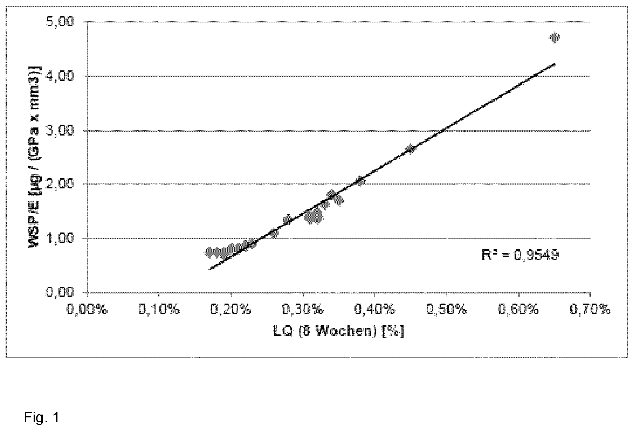

Die vorliegende Erfindung basiert auf eigenen Untersuchungen, die in der DE 10 2017 103 084.0 beschrieben sind. In diesen eigenen Untersuchen wurde erstmals gefunden, dass die lineare Quellung (LQ) einer indirekten Restauration proportional zur Wasseraufnahme ($W_{SP}$) und umgekehrt proportional zum Elastizitätsmodul (E) des Materials ist, vgl. Figur 1. Es gilt also folgende Beziehung:

$$LQ \sim \frac{W_{SP}}{E}$$

Diese Quellung führt bei einer Krone zu einer Ausdehnung und damit zur Vergrößerung des Außen- und Innendurchmessers, da sich die gesamte Restauration durch die Quellung vergrößert. Dabei beträgt die relative lineare Quellung (in Prozent) etwa ein Drittel der relativen Volumenquellung (in Prozent). Durch die Vergrößerung des Innendurchmessers entsteht eine Zugkraft, die auf den Haftverbund der befestigten Krone ausgeübt wird. Übersteigen die Zugkräfte lokal die Haftung, kommt es an diesen Stellen zu Abrissen des Befestigungsmaterials und zu Randspaltbildung. Diese Stellen bergen daher ein erhöhtes Risiko für die Ansiedlung von Bakterien und die Bildung von Sekundärkaries. Ist die Quellung groß genug oder die fortwährende Belastung der gebildeten Fehlstellen hoch genug, kommt es zum totalen Retentionsverlust der Restauration.

In dem Bestreben, retentionssicherere und randspaltfreie prothetische Versorgungen (indirekte dentale Restaurationen) zu erhalten, war es somit - anknüpfend an DE 10 2017 103 084.0 - eine spezifische Aufgabe der vorliegenden Erfindung, Fräsrohlinge aus Kunststoff oder einem kunststoffbasierten Komposit zur Verfügung zu stellen, die bei Wasserlagerung bzw. unter Bedingungen, wie sie in der Mundhöhle herrschen, möglichst quellfrei sind. Ausgehend von der technischen Lehre der DE 10 2017 103 084.0 und insbesondere den dort offenbarten dentalen Fräsrohlingen und Materialien war es eine spezifische Aufgabe der vorliegenden Erfindung, Fräsrohlinge und daraus hergestellte indirekte dentale Restauration zur Verfügung zu stellen, deren Fähigkeit zum Quellen noch einmal reduziert ist, die also weitgehend quellfrei sein sollen.

Ausgehend von der technischen Lehre der DE 10 2017 103 084.0 gingen erste Überlegungen im Rahmen der vorliegenden Erfindung in die Richtung von Materialien, deren Wasseraufnahme noch weiter reduziert ist oder deren Elastizitätsmodul besonders hoch ist. Durch solche Maßnahmen würde sich nämlich gemäß der vorstehend dargestellten Proportionalitätsbeziehung die lineare Quellung und damit vermutlich auch das Risiko eines Retentionsverlusts reduzieren lassen.

Die vorstehend genannten technischen Aufgaben wurden dann jedoch überraschenderweise auf eine vollständig andere Art und Weise gelöst.

Es wurde nämlich in eigenen Untersuchen überraschenderweise gefunden, dass sich die Retention von indirekten dentalen Restaurationen (insbesondere von mittels CAD/CAM-Verfahren aus einem Fräsrohling gefrästen Kronen) erheblich verbessern lässt, wenn Fräsrohlinge aus Kunststoff oder einem kunststoffbasierten Komposit, vorzugsweise solche, die selbst eine geringe Wasseraufnahmefähigkeit besitzen, einer gezielten Vorkonditionierung unterworfen werden, bei der die Fräsrohlinge Wasser aufnehmen.

Während bislang darauf abgestellt und versucht wurde, möglichst wenig Wasser in einen Fräsrohling aus Kunststoff oder einem kunststoffbasierten Komposit eindringen zu lassen (vgl. erneut die vorstehend angegebene Proportionalitätsbeziehung) wird im Rahmen der vorliegenden Erfindung und somit ganz entgegengesetzt zur Lehre gemäß DE 10 2017 103 084.0 ganz bewusst ein Fräsrohling erzeugt, der bereits Wasser in einer beträchtlichen Menge enthält. Dabei werden vorzugsweise Fräsrohlinge, die selbst bereits nur in geringem Maße zur Aufnahme von Wasser fähig sind, das heißt eine geringe Wasseraufnahme $W_{SP}$ (zur Bestimmungsmethode im Rahmen der vorliegenden Erfindung siehe unten) besitzen, eingesetzt und der besagten Vorkonditionierung unterworfen. Derart vorkonditionierte, das heißt mit Wasser infiltrierte und angereicherte Fräsrohlinge besitzen jeweils im Vergleich zu getrockneten Fräsrohlingen, wie sie z.B. im Testverfahren gemäß EN ISO 4049:2009 (D) vorliegen und durch Trocknen bis zur Gewichtskonstanz hergestellt werden, eine überraschenderweise nochmals entscheidend verringerte Quellfähigkeit.

Mit dem zusätzlichen Schritt der Vorkonditionierung gelingt es somit, die Quellfähigkeit von Fräsrohlingen aus Kunststoff oder einem kunststoffbasierten Komposit noch weiter zu reduzieren und so zu den Eigenschaften von Fräsrohlingen aus Keramik aufzuschließen. Zudem gelingt es durch den zusätzlichen Schritt der Vorkonditionierung, Werkstoffe (Kunststoffe bzw. kunststoffbasierte Komposite), die materialbedingt eine hohe Wasseraufnahme $W_{SP}$ und im getrockneten Zustand somit eine hohe Quellfähigkeit besitzen und daher als Material für indirekte dentale Restaurationen kaum geeignet sind, als Werkstoff für Fräsrohlinge zur Herstellung indirekter dentaler Restaurationen mit hervorragenden Retentionseigenschaften und Hafteigenschaften verfügbar zu machen.

Vorkonditionierte, d. h. mit Wasser behandelte und infiltrierte Fräsrohlinge aus Kunststoff oder einem kunststoffbasierten Komposit sind somit qualitativ höherwertig als die entsprechenden trockenen Fräsrohlinge und sie erhöhen auch die Zahl von Fräsrohlingtypen, die zur Herstellung indirekter dentaler Restaurationen gut geeignet sind, enorm. Vorkonditionierte, d. h. mit Wasser infiltrierte Fräsrohlinge aus Kunststoff oder einem kunstoffbasierten Kompositmaterial erreichen überraschenderweise die klinische Retentionsfestigkeit von Keramikblöcken, ohne dass sie deren Nachteile, wie die Neigung zu Frakturen sowie zum Chipping, besitzen. Zudem können Fräsrohlinge aus Kunststoff oder einem kunststoffbasierten Komposit sowie die daraus hergestellten indirekten dentalen Restaurationen bei Bedarf einfach und schnell repariert werden, im Gegensatz zu Fräsrohlingen aus Keramik bzw. den daraus hergestellten indirekten dentalen Restaurationen.

Mit Wasser vorkonditionierte Fräsrohlinge aus Kunststoff oder einem kunststoffbasierten Komposit sind im Stand der Technik nicht bekannt.

DE 699 22 413 T2 (Übersetzung der euroäischen Patentschrift EP 1 143 915 B1; 3M Innovative Properties Co.) offenbart dentale Fräsrohlinge, die (a) ein Polymerharz und (b) ein spezifisches, fein verteiltes Füllstoffmaterial enthalten, wobei der Rohling im Wesentlichen frei von Rissen ist und so gefertigt ist, dass der Rohling einen im Dokument näher spezifizierten Thermoschocktest besteht. Das Dokument lehrt, dass in manchen Fällen ein gehärteter Rohling einer spezifischen Hitzebehandlung unterzogen wird, und nach dem Abschluss der Hitzebehandlung kann der Rohling auf Raumtemperatur äquilibrieren, entweder durch Eintauchen in Wasser, das Raumtemperatur hat, oder durch langsames Kühlen über Umgebungstemperatur. Das Eintauchen in Wasser wird in dem Dokument nicht näher spezifiziert, da es aber lediglich der Temperaturäquilibrierung dienen soll, wird es nur für einen kurzen Zeitraum erfolgen; eine Vorkonditionierung im Sinne der vorstehenden Überlegungen findet somit aus physikalischen Gründen nicht statt. Es sei bereits an dieser Stelle auf die Tabellen 16 bis 18 des vorliegenden Textes verwiesen, aus denen sich ergibt, dass die Wasseraufnahme in Fräsrohlinge ein äußerst langsamer Prozess ist; selbst bei 37 °C (also einer Temperatur, die deutlich über Raumtemperatur liegt) dauert es mehrere Wochen, bis ein Konditionierungsgrad von 25 % erreicht wird; es versteht sich, dass bei Raumtemperatur die für eine Wasseraufnahme erforderlichen Transportprozesse noch signifikant langsamer ablaufen, so dass in für eine Temperaturäquilibrierung auf Raumtemperatur erforderlichen Zeiträumen überhaupt keine signifikante Wasseraufnahme erfolgen kann. Aus entsprechenden Gründen kommt es auch bei sonstigem kurzzeitigem Kontakt eines Fräsrohlings mit Wasser (z.B. beim Fräsen) nicht zu einer signifikanten Änderung des Wassergehalts.

Die Erfindung ist in den beigefügten Ansprüchen definiert.

Gemäß einem primären Aspekt betrifft die vorliegende Erfindung einen Fräsrohling zur Herstellung einer indirekten dentalen Restauration, aus Kunststoff oder einem kunststoffbasierten Komposit, umfassend Wasser in einer Menge von wenigstens 25 % der Wasseraufnahme $W_{SP}$.

Die Wasseraufnahme $W_{SP}$ ist dabei eine Eigenschaft eines Fräsrohlings, die vom tatsächlichen Wassergehalt unabhängig ist. Im Rahmen des vorliegenden Textes und im Zusammenhang mit der vorliegenden Erfindung wird zur Bestimmung der Wasseraufnahme $W_{SP}$ eine weiter unten angegebene Bestimmungsmethode verwandt, die sehr eng an die Vorgehensweise gemäß EN ISO 4049:2009 (D) angelehnt ist. Sofern nicht anders angegeben, beziehen sich sämtliche Angaben, insbesondere zu experimentell bestimmten Werten für die Wasseraufnahme $W_{SP}$, auf die weiter unten angegebene Bestimmungsmethode.

Zur Erläuterung: Im Rahmen dieser Bestimmungsmethode (und dies trifft analog für die Vorgehensweise gemäß EN ISO 4049:2009 (D) zu) wird ein Prüfkörper in einem ersten Schritt des Bestimmungsverfahrens bis zur Gewichtskonstanz getrocknet. Dann wird dieser getrocknete Prüfkörper unter definierten Bedingungen mit Wasser gesättigt und schließlich erneut getrocknet. Die Wasseraufnahme $W_{SP}$ ergibt sich - etwas vereinfacht ausgedrückt - aus (i) der Differenz der Masse des mit Wasser gesättigten und der Masse des anschließend getrockneten Prüfkörpers sowie (ii) dem Volumen des Prüfkörpers. Aufgrund dieser Vorgehensweise ist die Wasseraufnahme eines Prüfkörpers unabhängig davon, wie hoch oder niedrig der tatsächliche Wassergehalt vor dem ersten Trocknen war. Die "Wasseraufnahme $W_{SP}$" ist nach ihrer Art ein Wert, der die theoretisch maximal in einem Prüfkörper enthaltene Wassermenge auf die Masse des getrockneten Prüfkörpers bezieht.

Ein erfindungsgemäßer Fräsrohling besteht aus Kunststoff oder einem kunststoffbasierten Komposit. Der Begriff "Fräsrohling aus Kunststoff bezeichnet dabei solche Fräsrohlinge, die keinen Füllstoff umfassen, der Begriff "Fräsrohling aus kunststoffbasiertem Komposit" bezeichnet im Unterschied dazu Fräsrohlinge, die aus einem Komposit bestehen, welches eine Harzmatrix (d. h. eine Kunststoffmatrix) sowie darin dispergiert einen Füllstoff, vorzugsweise einen anorganischen Füllstoff, umfassen.

Erfindungsgemäße Fräsrohlinge umfassen Wasser in einer Menge von wenigstens 25 % der Wasseraufnahme $W_{SP}$. Zur Bestimmung der Wasseraufnahme $W_{SP}$ sei auf die vorstehenden Ausführungen sowie auf die Bestimmungsmethode verwiesen, die weiter unten angegeben ist.

Ein gegebener Fräsrohling mit zunächst unbekanntem Wassergehalt umfasst zumindest dann Wasser in einer Menge von wenigstens 25 % der Wasseraufnahme $W_{SP}$, wenn er beim Trocknen bis zur Massenkonstanz unter den weiter unten angegebenen sonstigen Bedingungen eine Masse pro Volumeneinheit (z.B. pro Milliliter) verliert, die wenigstens 25 % der Masse pro Volumeneinheit entspricht, die beim Trocknen eines Vergleichs-Fräsrohlings verloren wird, der zuvor bis zur Massenkonstanz in Wasser gelagert worden war.

Ein erfindungsgemäßer Fräsrohling wie vorstehend und in den beigefügten Patentansprüchen definiert umfasst Wasser in einer Menge von wenigstens 25% der Wasseraufnahme $W_{SP}$ (zur Bestimmungsmethode vgl. die obigen Anmerkungen und die nachfolgenden Beispiele). Bevorzugt ist jedoch ein erfindungsgemäßer Fräsrohling umfassend Wasser in einer Menge von wenigstens 50%, bevorzugt wenigstens 75%, besonders bevorzugt wenigstens 90% der Wasseraufnahme $W_{SP}$. Es hat sich in eigenen Untersuchungen gezeigt, dass die Retentionseigenschaften und das Haftverhalten mit zunehmendem Wassergehalt eines Fräsrohlings fortschreitend positiv beeinflusst werden. Der Fachmann wird einen erfindungsgemäßen Fräsrohling hinsichtlich der in ihm enthaltenen Wassermenge so einstellen, wie es der Einzelfall erfordert. Falls aus einem Fräsrohling ganz spezifische indirekte dentale Restaurationen gefräst werden, also beispielsweise Inlays, Onlays, Teilkronen, Kronen, Veneers (Verblendungen) oder Brücken, so bestehen hinsichtlich der Retention und der Haftung im Einzelfall unterschiedliche Anforderungen, die der Fachmann berücksichtigt. Sofern an die Retention und die Haftung äußerst hohe Ansprüche gestellt werden, wird der Fachmann regelmäßig einen erfindungsgemäßen Fräsrohling präferieren, der Wasser in einer Menge von wenigstens 90% der Wasseraufnahme $W_{SP}$ umfasst.

Entscheidend ist hinsichtlich des Aspekts der Haftungseigenschaften, dass eine indirekte dentale Restauration unter den in der Mundhöhle herrschenden Bedingungen auch nach längerer Zeit noch gut anhaftet. In den Beispielen weiter unten werden solche Bedingungen nachgestellt durch einen Test, bei dem eine idealisierte Krone auf einem idealisierten Abutment befestigt wird und anschließend das resultierende Ensemble für 8 Wochen bei 37°C gelagert wird. Erfindungsgemäße Fräsrohlinge schneiden in derartigen Untersuchungen zur Haftung immer deutlich besser ab als Vergleichs-Fräsrohlinge, die Wasser in einer Menge von deutlich weniger als 25% der Wasseraufnahme $W_{SP}$ enthalten. Die besten Resultate werden mit erfindungsgemäßen Fräsrohlingen erhalten, die Wasser in einer Menge von wenigstens 90% der Wasseraufnahme $W_{SP}$ enthalten.

Wenngleich erfindungsgemäße Fräsrohlinge im jeweiligen Vergleich mit Fräsrohlingen, die Wasser in einer Menge von deutlich weniger als 25% der Wasseraufnahme $W_{SP}$ enthalten, deutlich verbesserte Eigenschaften besitzen (insbesondere eine verbesserte Retention und eine verbesserte Haftung nach Befestigung und Wasserlagerung) ist es zum Erreichen von besonders vorteilhaften Absolutwerten sinnvoll, im Sinne von an Dokument DE10 2017 103 084.0 anknüpfenden Überlegungen auch die sonstigen physikalischen Parameter eines erfindungsgemäßen Fräsrohlings vorteilhaft einzustellen.

Bevorzugt ist daher ein erfindungsgemäßer Fräsrohling, vorzugsweise wie vorstehend als bevorzugt bezeichnet, umfassend Wasser in einer Menge von wenigstens 90% der Wasseraufnahme $W_{SP}$, mit einem E-Modul, bestimmt nach der ADA Specification No. 27 - 1993, von wenigstens 10 GPa, vorzugsweise von wenigstens 13 GPa und besonders bevorzugt von wenigstens 15 GPa. Der Fachmann wird den angestrebten E-Modul eines erfindungsgemäßen Fräsrohlings in üblicher Weise durch Auswahl von Art und Menge der eingesetzten Füllstoffe und Monomeren sowie ggf. durch den gewählten Aushärtungsmechanismus und dergleichen einstellen.

Insoweit ist zu berücksichtigen, dass eigene Untersuchungen gezeigt haben, dass der E-Modul eines Fräsrohlings nicht signifikant von der Menge des in ihm enthaltenen Wassers abhängt. Wie vorstehend ausgeführt, verhält sich die lineare Quellung (LQ) einer indirekten dentalen Restauration gemäß eigenen Untersuchungen in etwa umgekehrt proportional zum E-Modul. Da die Retentionseigenschaften und die Hafteigenschaften regelmäßig direkt abhängig von der linearen Quellung sind, erweisen sich erfindungsgemäße Fräsrohlinge mit einem hohen E-Modul (wie vorstehend ausgeführt) als besonders vorteilhaft.

Vorzugsweise besitzt ein erfindungsgemäßer Fräsrohling, vorzugsweise ein erfindungsgemäßer Fräsrohling wie vorstehend als bevorzugt bezeichnet, insbesondere also ein erfindungsgemäßer Fräsrohling umfassend Wasser in einer Menge von wenigstens 90% der Wasseraufnahme $W_{SP}$ und/oder mit einem E-Modul von wenigstens 10 GPa, bevorzugt wenigstens 15 GPa, eine Wasseraufnahme $W_{SP}$ von höchstens 40 $\mu g/mm^3$, vorzugsweise von höchstens 30 $\mu g/mm^3$ und besonders bevorzugt von höchstens 20 $\mu g/mm^3$.

Wie vorstehend ausgeführt, verhält sich die lineare Quellung in etwa proportional zur Wasseraufnahme $W_{SP}$ (wie oben definiert; zur Bestimmungsmethode siehe unten). Erfindungsgemäße Fräsrohlinge mit einer Wasseraufnahme von höchstens 40 $\mu g/mm^3$, vorzugsweise von höchstens 30 $\mu g/mm^3$ und besonders bevorzugt von höchstens 20 $\mu g/mm^3$ zeigen daher besonders hervorragende Retentionseigenschaften und Hafteigenschaften (insbesondere nach der Befestigung und nach Wasserlagerung bzw. in der Mundhöhle).

Besonders bevorzugt sind erfindungsgemäße Fräsrohlinge zu Herstellung einer indirekten dentalen Restauration (aus Kunststoff oder einem kunststoffbasierten Komposit), umfassend Wasser in einer Menge von wenigstens 90% der Wasseraufnahme $W_{SP}$, mit einem E-Modul von wenigstens 13 GPa (bevorzugt wenigstens 15 GPa) und einer Wasseraufnahme $W_{SP}$ von höchstens 20 $\mu g/mm^3$.

Angesichts des in eigenen Untersuchungen ermittelten Zusammenhangs zwischen der linearen Quellung und dem Quotienten aus der Wasseraufnahme $W_{SP}$ und dem E-Modul E sind erfindungsgemäße Fräsrohlinge besonders bevorzugt, bei denen der Quotient aus der Wasseraufnahme $W_{SP}$ (wie vorstehend definiert; zur Bestimmungsmethode siehe unten) und dem nach der ADA Specification No. 27 - 1993 bestimmten E-Modul kleiner ist als 1,35 $\mu g/(GPa \times mm^3)$, vorzugsweise kleiner ist als 1,00 $\mu g/(GPa \times mm^3)$.

Eigene Untersuchungen, die weiter unten in den Beispielen zusammenfassend dargestellt sind, belegen, dass für die bevorzugten erfindungsgemäßen Fräsrohlinge (mit bevorzugten E-Moduln und Wasseraufnahmen sowie Quotienten aus Wasseraufnahme $W_{SP}$ und E-Modul) besonders hervorragende Ergebnisse erzielt werden, insbesondere für die Haftung nach Befestigung und Wasserlagerung.

Besonders bevorzugt ist ein erfindungsgemäßer Fräsrohling zur Herstellung einer indirekten dentalen Restauration aus Kunststoff oder einem kunststoffbasierten Komposit, umfassend Wasser in einer Menge von wenigstens 50%, bevorzugt wenigstens 75%, besonders bevorzugt wenigstens 90% der Wasseraufnahme $W_{SP}$ und/oder mit einem E-Modul, bestimmt nach der ADA Specification No. 27 - 1993, von wenigstens 10 GPa, vorzugsweise von wenigstens 13 GPa und besonders bevorzugt von wenigstens 15 GPa und/oder mit einer Wasseraufnahme $W_{SP}$ von höchstens 40 $\mu g/mm^3$, vorzugsweise von höchstens 30 $\mu g/mm^3$ und besonders bevorzugt von höchstens 20 $\mu g/mm^3$ und/oder wobei der Quotient aus der Wasseraufnahme $W_{SP}$ und dem nach der ADA Specification No. 27 - 1993 bestimmten E-Modul kleiner ist als 1,35 $\mu g/(GPa \times mm^3)$, vorzugsweise kleiner ist als 1,00 $\mu g/(GPa \times mm^3)$; vorzugsweise gilt hier jeweils die "und"-Verknüpfung. Zudem sind vorzugsweise zwei oder mehr der jeweils anspruchsvollsten (engsten) Definitionen miteinander verknüpft (90% Wasseraufnahme $W_{SP}$; E-Modul von wenigstens 15 GPa; Wasseraufnahme $W_{SP}$ von höchstens 20 $\mu g/mm^3$; Quotient vorzugsweise kleiner als 1,00 $\mu g/GPa \times mm^3$). Vorzugsweise sind sämtliche dieser anspruchsvollsten (engsten) Definitionen miteinander verknüpft. Entsprechende Fräsrohlinge sind aufgrund ihrer ganz hervorragenden Retentionseigenschaften und Haftungseigenschaften besonders bevorzugt.

Ganz besonders bevorzugt ist ein erfindungsgemäßer Fräsrohling wie vorstehend definiert (vorzugsweise ein erfindungsgemäßer Fräsrohling, wie er vorstehend als bevorzugt oder besonders bevorzugt bezeichnet ist), wobei die Differenz zwischen der Wasseraufnahme $W_{SP}$ und dem Wassergehalt des Fräsrohlings (bestimmt durch Trocknen unter den weiter unten angegebenen Bedingungen und Differenzwägung sowie Volumenbestimmung) kleiner ist als 10 $\mu g/mm^3$, vorzugsweise kleiner als 8 $\mu g/mm^3$ bevorzugt kleiner ist als 4 $\mu g/mm^3$.

Die besagte Differenz entspricht der nach Vorkonditionierung verbleibenden Wasseraufnahmefähigkeit des vorkonditionierten erfindungsgemäßen Fräsrohlings. Ein Fräsrohling, für den die Differenz auf einen Wert von kleiner 10 $\mu g/mm^3$ bestimmt ist, kann zumindest bei einer Temperatur von 37°C nur noch weniger als 10 $\mu g$ Wasser pro $mm^3$ Fräsrohling aufnehmen. Dementsprechend ist ein solcher bevorzugter erfindungsgemäßer Fräsrohling kaum noch quellfähig. Aus einem solchen Fräsrohling hergestellte Kronen erreichen in entsprechenden Tests nach Befestigung auf einem Abutment auch nach langer Wasserlagerung hervorragende Haftungswerte.

Es ist eine besondere Leistung der vorliegenden Erfindung, (vorkonditionierte) Fräsrohlinge anzugeben, die (zusätzlich) nicht mehr als 10 $\mu g$ Wasser pro $mm^3$ Fräsrohling (vorzugsweise weniger als 8 $\mu g/mm^3$, weiterbevorzugt weniger als 4 $\mu g/mm^3$) aufnehmen können. Derartige Fräsrohlinge sind aus dem Stand der Technik nicht bekannt.

Ist die Wasseraufnahme $W_{SP}$ eines gegebenen Fräsrohlings groß, so muss einem solchen Fräsrohling eine beträchtliche Menge an Wasser zugeführt werden, damit die besagte Differenz kleiner wird als 10 $\mu g/mm^3$ (vorzugsweise kleiner als 8 $\mu g/mm^3$, bevorzugt kleiner als 4 $\mu g/mm^3$). Ist die Wasseraufnahme $W_{SP}$ eines gegebenen Fräsrohlings hingegen bereits sehr klein (und liegt beispielsweise bei 12 $\mu g/mm^3$, vgl. für dieses Beispiel etwa Beispiel 4 unten), dann muss dieser Fräsrohling nur vergleichsweise geringe Mengen an Wasser aufnehmen, damit die Differenz zwischen der Wasseraufnahme $W_{SP}$ und dem Wassergehalt des Fräsrohlings kleiner wird als 10 $\mu g/mm^3$, vorzugsweise kleiner als 8 $\mu g/mm^3$, bevorzugt kleiner als 4 $\mu g/mm^3$.

Besonders bevorzugt ist ein erfindungsgemäßer Fräsrohling aus kunststoffbasiertem Komposit, umfassend

a) einen anorganischen Füllstoff in einer Menge von wenigstens 70 Gew.-%, vorzugsweise wenigstens 80 Gew.-%, bezogen auf die Gesamtmasse des Fräsrohlings und

b) eine Harzmatrix (Kunststoffmatrix).

Neben dem anorganischen Füllstoff und der Harzmatrix können in einem solchen bevorzugten erfindungsgemäßen Fräsrohling noch Spuren von Initiatoren für die Härtung sowie sonstige Additive enthalten sein. Ein erfindungsgemäßer Fräsrohling aus kunststoffbasiertem Komposit, der einen anorganischen Füllstoff in der angegebenen bevorzugten Menge sowie eine Harzmatrix umfasst, ist hinsichtlich der enthaltenen Menge an Wasser, dem E-Modul, der Wasseraufnahme $W_{SP}$ und/oder dem Quotienten aus der Wasseraufnahme $W_{SP}$ und dem E-Modul vorzugsweise so ausgestaltet, wie es vorstehend als bevorzugt angegeben ist.

Vorzugsweise ist ein bevorzugter erfindungsgemäßer Fräsrohling aus kunststoffbasiertem Komposit, der einen anorganischen Füllstoff in der vorstehend angegebenen Menge sowie eine Harzmatrix umfasst, so ausgestaltet, dass der anorganische Füllstoff a) umfasst:

a1) eine Glaszusammensetzung und

a2) nicht aggregierte und nicht agglomerierte Kieselsäure mit einer mittleren Teilchengröße von nicht mehr als 80 nm (zur Messmethode siehe unten).

Besonders vorteilhaft ist insoweit ein erfindungsgemäßer Fräsrohling, wobei die Glaszusammensetzung a1)

eine erste Glaszusammensetzung a1a) mit einem D50-Wert im Bereich von 0,4 bis 1,0 $\mu m$, vorzugsweise im Bereich von 0,5 bis 0,9 $\mu m$,

und

eine zweite Glaszusammensetzung a1b) mit einem D50-Wert im Bereich von 1,2 bis 5,0 $\mu$m, vorzugsweise im Bereich von 1,5 bis 4,0 $\mu$m,

umfasst,

wobei das Massenverhältnis von a1a) zu a1b) zwischen 1 : 1,5 und 1 : 8, vorzugsweise zwischen 1 : 2 und 1 : 5 liegt,

wobei das Massenverhältnis von a2) zur Summe von a1a) und a1b) zwischen 1 : 3 und 1 : 6 liegt,

wobei das Verhältnis des D50-Werts der ersten Glaszusammensetzung a1a) zum D50-Wert der zweiten Glaszusammensetzung a1b) im Bereich von 1 : 1,5 bis 1 : 10, vorzugsweise 1 : 2 bis 1 : 5, liegt,

und wobei der D75-Wert der ersten Glaszusammensetzung a1a) kleiner ist als der D25-Wert der zweiten Glaszusammensetzung a1b).

In erfindungsgemäßen Fräsrohlingen aus kunststoffbasiertem Komposit, die ein anorganischen Füllstoff a) und eine Harzmatrix b) umfassen, kann die Harzmatrix bis zu 30 Gew.-% ausmachen, bezogen auf die Gesamtmasse des Fräsrohlings. Es sind jedoch häufig neben den Bestandteilen anorganischer Füllstoff a) und Harzmatrix b) auch ein oder mehrere weitere Bestandteile vorgesehen, die weder dem anorganischen Füllstoff noch der Harzmatrix zugerechnet werden, beispielsweise Reste von Initiatoren für die Härtung sowie sonstige Additive einschließlich etwaiger organischer Füllstoffe.

Ein bevorzugter erfindungsgemäßer Fräsrohling besteht aus einem kunststoffbasierten Komposit und umfasst

a) einen anorganischen Füllstoff in einer Menge von wenigstens 70 Gew.-%, vorzugsweise wenigstens 80 Gew.-%, bezogen auf die Gesamtmasse des Fräsrohlings.
und

b) eine Harzmatrix,

wobei der anorganische Füllstoff a) umfasst:

a1) eine Glaszusammensetzung und

a2) nicht aggregierte und nicht agglomerierte Kieselsäure mit einer mittleren Teilchengröße von nicht mehr als 80 nm,

wobei die Glaszusammensetzung a1)

eine erste Glaszusammensetzung a1a) mit einem D50-Wert im Bereich von 0,4 bis 1,0 $\mu$m, vorzugsweise im Bereich von 0,5 bis 0,9 $\mu$m,

und

eine zweite Glaszusammensetzung a1b) mit einem D50-Wert im Bereich von 1,2 bis 5,0 $\mu$m, vorzugsweise im Bereich von 1,5 bis 4,0 $\mu$m,

umfasst,

wobei das Massenverhältnis von a1a) zu a1b) zwischen 1 : 1,5 und 1 : 8, vorzugsweise zwischen 1 : 2 und 1 : 5 liegt,

wobei das Massenverhältnis von a2) zur Summe von a1a) und a1b) zwischen 1 : 3 und 1 : 6 liegt,

wobei das Verhältnis des D50-Werts der ersten Glaszusammensetzung a1a) zum D50-Wert der zweiten Glaszusammensetzung a1b) im Bereich von 1 : 1,5 bis 1 : 10, vorzugsweise 1 : 2 bis 1 : 5, liegt,

und wobei der D75-Wert der ersten Glaszusammensetzung a1a) kleiner ist als der D25-Wert der zweiten Glaszusammensetzung a1b).

Besonders bevorzugt sind hierbei Ausgestaltungen, bei denen mehrere oder sämtliche der anspruchsvollsten (engsten) Definitionen miteinander verknüpft sind, das heißt: wenigstens 80 Gew.-% anorganischer Füllstoff; D50-Wert der ersten Glaszusammensetzung a1a) im Bereich von 0,5 bis 0,9 $\mu$m; D50-Wert der zweiten Glaszusammensetzung a1b) im Bereich von 1,5 bis 4,0 $\mu$m; Massenverhältnis von a1a) zu a1b) zwischen 1 : 2 und 1 : 5; Verhältnis des D50-Werts der ersten Glaszusammensetzung a1a) zum D50-Wert der zweiten Glaszusammensetzung a1b) im Bereich von 1 : 2 bis 1 : 5. Es versteht sich, dass diese bevorzugten Ausgestaltungen vorzugsweise auch hinsichtlich des Wassergehalts (bevorzugt wenigstens 90% der Wasseraufnahme $W_{SP}$), des E-Moduls (bevorzugt wenigstens 15 GPa), der Wasseraufnahme $W_{SP}$ (bevorzugt höchstens 20 $\mu$g/mm$^3$) und/oder dem Quotienten aus Wasseraufnahme $W_{SP}$ und E-Modul (vorzugsweise kleiner als 1,00 pg/GPa x mm$^3$) in der bevorzugten Weise eingestellt bzw. ausgewählt sind.

Bevorzugt ist ein erfindungsgemäßer Fräsrohling, der a) einen anorganischen Füllstoff in einer Menge von wenigstens 70 Gew.-%, vorzugsweise wenigstens 80 Gew.-%, bezogen auf die Gesamtmasse des Fräsrohlings und b) eine Harzmatrix umfasst, wobei die Harzmatrix b) ein Polymerisat von Monomeren ist, die difunktionelle (Meth)acrylate enthalten, wobei der Gewichtsanteil von ethoxyliertem Bisphenol-A-Dimethacrylat mit einem durchschnittlichen Ethoxylierungsgrad von 2 bis 4 Ethoxygruppen pro Molekül größer ist als 40 Gew.-% und kleiner ist als 50 Gew.-%, bezogen auf die Gesamtmasse der Monomere.

Ein solcher Fräsrohling wird unter Verwendung einer Monomerenmischung hergestellt, die difunktionelle (Meth)acrylate enthält, wobei der Gewichtsanteil von ethoxyliertem Bisphenol-A-Dimethacrylat mit einem durchschnittlichen Ethoxylierungsgrad von 2 bis 4 Ethoxygruppen pro Molekül in der Monomerenmischung größer ist als 40 Gew.-% und kleiner ist als 50 Gew.-%, bezogen auf die Gesamtmasse der Monomere. Nach Vermischen der Monomerenmischung, eines oder mehrerer Initiatoren für die Härtung sowie optional vorhandener Additive mit der erforderlichen Menge von anorganischem Füllstoff erfolgt die Härtung der Monomerenmischung durch Polymerisation zur Harzmatrix b) in üblicher Weise, z.B. durch Strahlenhärtung (photochemisch) und/oder durch chemische Härtung (Redoxreaktion) und/oder thermisch.

Der Einsatz von ethoxyliertem Bisphenol-A-dimethacrylat mit einem durchschnittlichen Ethoxylierungsgrad von 2 bis 4 Ethoxygruppen pro Molekül in der angegebenen Menge führt zu besonders guten Ergebnissen in Untersuchungen zur Haftung und in der Praxis zu einer besonders guten Retention von entsprechenden indirekten dentalen Restaurationen. Hinsichtlich eines Vergleiches von Eigenschaften erfindungsgemäßer Fräsrohlinge in Abhängigkeit vom Ethoxylierungsgrad eingesetzter Mengen von ethoxyliertem Bisphenol-A-dimethacrylat sei auf die Beispiele 9 bis 12 und die zugeordneten Tabellen 6 und 7 verwiesen.

Ein erfindungsgemäßer Fräsrohling ist zur Herstellung einer indirekten dentalen Restauration geeignet und vorgesehen. Vorzugsweise sind die Abmessungen eines erfindungsgemäßen Fräsrohlings so gewählt, dass aus ihm

- ein Würfel mit einer Kantenlänge von 10 mm, vorzugsweise 14 mm,
  und/oder

- ein Quader mit einer quadratischen Grundfläche mit einer Kantenlänge von 10mm, vorzugsweise 14 mm, und einer Höhe von 20 mm
  fräsbar ist.

Entsprechend dimensionierte erfindungsgemäße Fräsrohlinge sind dazu geeignet, bei der Herstellung von Inlays, Onlays, Teilkronen, Kronen, Veneers (Verblendungen) und Brücken eingesetzt zu werden.

Es sei darauf hingewiesen, dass in der industriellen Praxis bislang etwaige Laboruntersuchungen zur Wasseraufnahme etc. regelmäßig nicht an Fräsrohlingen durchgeführt wurden, sondern an daraus hergestellten Prüfkörpern deutlich geringerer Abmessungen, z.B. dünnen Scheiben. Solche Prüfkörper sind regelmäßig keine Fräsrohlinge im Sinne der vorliegenden Erfindung, weil sie nicht zur Herstellung einer indirekten dentalen Restauration geeignet sind, und sie besitzen nicht die bevorzugten Abmessungen, wie sie vorstehend angegeben sind.

Nachfolgend werden bevorzugte Bestandteile erfindungsgemäßer Fräsrohlinge bzw. von aushärtbaren Mischungen angegeben, aus denen erfindungsgemäße Fräsrohlinge herstellbar sind.

a) anorganische Füllstoffe:

Der erfindungsgemäße Fräsrohling umfasst anorganische Füllstoffe in einer Menge von wenigstens 70 Gew.-%, vorzugsweise wenigstens 80 Gew.-%, bezogen auf die Gesamtmasse des Fräsrohlings, und dementsprechend enthalten aushärtbare Mischungen zur Herstellung eines erfindungsgemäßen Fräsrohlings anorganische Füllstoffe in einer Menge von mindestens 70 Gew.-%, vorzugsweise von mindestens 80 Gew.-%, bezogen auf die Gesamtzusammensetzung der Mischung. Anorganische Füllstoffe werden vorzugsweise als Mischung diverser Füllstofffraktionen eingesetzt; zur Optimierung der Produkteigenschaften werden anorganische Füllstoffe in unterschiedlichen Korngrößen in die Rezepturen eingebracht, wobei sie vorzugsweise eine multimodale, ganz bevorzugt eine bimodale Verteilung aufweisen.

Abhängig von den Bedürfnissen des Einzelfalls werden anorganische Füllstoffe in Form von kompakten Gläsern und/oder in Form unterschiedlicher Kieselsäuren in verschiedenen Größen und Zuständen (monodispers, polydispers) als Bestandteile erfindungsgemäßer Fräsrohlinge und entsprechender Vor-Mischungen präferiert.

Geeignete anorganische Bestandteile sind beispielsweise amorphe Materialien auf der Basis von Mischoxiden aus $SiO_2$, $ZrO_2$ und/oder $TiO_2$ sowie Füllstoffe wie Quarz-Glaskeramik oder Glaspulver, Bariumsilikatgläser, Bariumfluorsilikatgläser, Strontiumsilikatgläser, Strontiumborsilikat, Li/Al-Silikatgläser, Bariumgläser, Calciumsilikate, Natriumaluminiumsilikate, Fluoraluminiumsilikatgläser, Oxide von Aluminium oder Silicium, Zeolithe, Apatit, Zirkonsilikate, schwerlösliche Metallsalze wie Bariumsulfat oder Calciumfluorid sowie röntgenopake Füllstoffe wie Ytterbiumfluorid.

Vorzugsweise enthält ein erfindungsgemäßer Fräsrohling Barium-Aluminium-Borsilikatgläser als Bestandteil der Füllstoffkomponente a).

Zum besseren Einbau in die Harzmatrix (Kunststoffmatrix; Polymermatrix) können die genannten Materialien organisch oberflächenmodifiziert sein; dies ist in vielen Fällen bevorzugt. Beispielhaft genannt sei die Oberflächenbehandlung anorganischer Füllstoffe mit einem Silan. Als Haftvermittler eignet sich besonders das Methacryloxypropyltrimethoxysilan.

Vorzugsweise enthält ein erfindungsgemäßer Fräsrohling oberflächenbehandelte Barium-Aluminium-Borsilikatgläser, vorzugsweise silanisierte Barium-Aluminium-Borsilikatgläser und am meisten bevorzugt mit Methacryloxypropyltrimethoxysilan behandelte Barium-Aluminium-Borsilikatgläser.

In erfindungsgemäßen Fräsrohlingen werden vorzugsweise und in Abhängigkeit von den Erfordernissen des Einzelfalls unterschiedliche Kieselsäuren eingesetzt.

Vorzugsweise, wie vorstehend im Zusammenhang mit dem Bestandteil a2) ausgeführt, enthalten erfindungsgemäße Fräsrohlinge nanoskalige Kieselsäuren, das heißt Kieselsäurepartikel mit einer mittleren Teilchengröße von nicht mehr als 80 nm. Diese Kieselsäuren sind vorzugsweise nicht aggregiert und nicht agglomeriert. Die Herstellung der nanoskaligen Kieselsäuren erfolgt auf bekannte Weise, zum Beispiel durch Flammpyrolyse, Plasmaverfahren, Gasphasenkondensation, Kolloidtechniken, Präzipitationsverfahren, Sol-Gel Verfahren, etc.

Sofern die nanoskaligen Kieselsäuren in nicht agglomerierter und nicht aggregierter Form vorliegen, liegen Sie vorzugsweise in monodisperser Form vor. Dies ist besonders bevorzugt. Um eine gute Einbindung der Nanopartikel (Partikel mit einer mittleren Teilchengröße von nicht mehr als 80nm) in die Harzmatrix (Polymermatrix; Kunststoffmatrix) einer radikalisch härtbaren dentalen Zusammensetzung zur Herstellung eines erfindungsgemäßen Fräsrohlings zu ermöglichen, sind die Oberflächen der nanoskaligen Kieselsäuren vorzugsweise organisch oberflächenmodifiziert, das heißt ihre Oberflächen weisen organische Strukturelemente auf. Beispielhaft genannt sei erneut die Oberflächenbehandlung der Füllstoffe mit einem Silan. Als Haftvermittler eignet sich auch für die besagten Nanopartikel besonders das Methacryloxypropyltrimethoxysilan.

Ein erfindungsgemäßer Fräsrohling enthält besonders bevorzugt oberflächenbehandelte nanoskalige, nicht agglomerierte und nicht aggregierte Kieselsäureteilchen mit einer mittleren Partikelgröße von nicht mehr als 80 nm, vorzugsweise silanisierte nanoskalige, nicht agglomerierte und nicht aggregierte Teilchen mit einer mittleren Partikelgröße von nicht mehr als 80 nm und am meisten bevorzugt mit Methacryloxypropyltrimethoxysilan behandelte, nanoskalige, nicht agglomerierte und nicht aggregierte Kieselsäureteilchen mit einer mittleren Partikelgröße von nicht mehr als 80 nm.

Kommerziell erhältliche nanoskalige, nicht agglomerierte und nicht aggregierte Kieselsole, die bei Herstellung eines erfindungsgemäßen Fräsrohlings vorzugsweise eingesetzt werden können, sind beispielsweise unter der Bezeichnung "NALCO COLLOIDAL SILICAS" (Nalco Chemical Co.), "Ludox colloidal silica" (Grace) oder "Highlink OG (Clariant) im Handel.

Vorzugsweise umfasst der Füllstoffanteil eines erfindungsgemäßen Fräsrohlings eine Mischung von a2) nicht aggregierter und nicht agglomerierter Kieselsäure mit einer mittleren Teilchengröße von nicht mehr als 80 nm und einem zweiten Füllstoff in Form von Mikropartikeln mit einer mittleren Partikelgröße im Bereich von 0,4 μm bis 5 μm. Bei diesem zweiten Füllstoff handelt es sich vorzugsweise um die vorstehend als Komponente a1) definierte Glaszusammensetzung eines erfindungsgemäßen Fräsrohlings. Durch die Kombination von Nanopartikeln, das heißt nicht aggregierter und nicht agglomerierter Kieselsäure mit einer mittleren Teilchengröße von nicht mehr als 80 nm mit Mikropartikeln (vorzugsweise Mikropartikeln einer Glaszusammensetzung, vgl. a1) oben) wird eine besonders vollständige und gleichmäßige Volumenfüllung des erfindungsgemäßen Fräsrohlings erreicht.

Innerhalb eines entsprechenden erfindungsgemäßen Fräsrohlings bewirken die Mikropartikel eine weitgehende gleichmäßige Füllung des Volumens, wobei die verbleibenden Hohlräume zwischen den Mikropartikeln durch die oben beschriebenen Nanopartikel (Komponente a2)) zumindest teilweise gefüllt werden. Unter Mikropartikeln werden im Zusammenhang mit der vorliegenden Erfindung Partikel mit einer mittleren Partikelgröße von 400 nm bis 5 μm verstanden. Der Einsatz von Glaszusammensetzungen als Mikropartikel ist bevorzugt.

Sofern im anorganischen Füllstoff a) eines bevorzugten erfindungsgemäßen Fräsrohlings Mikropartikel enthalten sind (vorzugsweise Mikropartikel einer Glaszusammensetzung a1)) weisen diese Mikropartikel vorzugsweise eine bimodale Partikelgrößenverteilung auf. Mikropartikel mit einer bimodalen Partikelgrößenverteilung sind bevorzugt, da mit ihnen eine vollständigere Volumenfüllung erreichbar ist als bei Verwendung von Mikropartikeln mit monomodaler Partikelgrößenverteilung. Bei Vorliegen einer bimodalen Partikelgrö-ßenverteilung bewirken die Partikel der Fraktionen mit der größeren Partikelgröße eine grobe Ausfüllung des Volumens, während die Partikel der Fraktion mit der kleineren Partikelgröße soweit möglich die Bereiche zwischen den Partikeln der Fraktionen mit der größeren Partikelgröße ausfüllen werden. Die dann noch verbleibenden Hohlräume werden wie oben beschrieben durch Nanopartikel gefüllt.

Bevorzugt ist die Verwendung einer Mischung zweier Mikropartikelfraktionen, wobei eine erste Mikropartikelfraktion einen D50-Wert im Bereich von 0,4 bis 1,0 $\mu$m, vorzugsweise im Bereich von 0,5 bis 0,9 $\mu$m besitzt. Vorzugsweise handelt es sich hierbei um eine erste Glaszusammensetzung a1a) (siehe für bevorzugte Ausgestaltungen oben). Die zweite Mikropartikelfraktion besitzt einen D50-Wert im Bereich von 1,2 $\mu$m bis 5,0 $\mu$m, vorzugsweise im Bereich von 1,5 $\mu$m bis 4,0 $\mu$m. Vorzugsweise handelt es sich hierbei um eine zweite Glaszusammensetzung a1b) wie oben definiert (für bevorzugte Ausgestaltungen siehe oben).

Vorzugsweise liegt das Verhältnis der Gesamtmasse einer solchen ersten Mikropartikelfraktion zur Gesamtmasse einer solchen zweiten Mikropartikelfraktion im Bereich von 1 : 1,5 bis 1 : 8, vorzugsweise im Bereich von 1 : 2 bis 1 : 5. Dies gilt insbesondere dann, wenn die erste Mikropartikelfraktion eine erste Glaszusammensetzung a1a) ist und die zweite Mikropartikelfraktion eine zweite Glaszusammensetzung a1b) ist.

b) Harzmatrix (Kunststoffmatrix, Polymermatrix) sowie Monomere zur Herstellung einer solchen Harzmatrix:

Ein erfindungsgemäßer Fräsrohling zur Herstellung einer indirekten dentalen Restauration bestehend aus Kunststoff oder einem kunststoffbasierten Komposit. Zur Herstellung des gehärteten Kunststoffs bzw. der Harzmatrix (Kunststoffmatrix; Polymermatrix, welche den Kunststoffanteil im kunststoffbasierten Komposit ausmacht), werden radikalisch polymerisierbare Mononmere als Bestandteil einer radikalisch aushärtbaren Zusammensetzung eingesetzt, die daneben noch anorganischen Füllstoff der Komponente a) sowie gegebenenfalls weitere Komponenten enthält. Der Anteil des Polymerisats der radikalisch polymerisierbaren Monomere in einem erfindungsgemäßen Fräsrohling ist vorzugsweise nicht höher als 30 Gew.-%, denn bevorzugt ist ein anorganischer Füllstoff in einer Menge von wenigstens 70 Gew.-% anwesend (siehe oben). Für die radikalisch härtbare Zusammensetzung, in der die radikalisch polymerisierbaren Monomere neben Füllstoffen eingesetzt werden, gilt entsprechendes.

Die radikalisch polymerisierbaren Monomere sind vorzugsweise die in der Dentalchemie üblicherweise in Kompositmaterialien eingesetzten (Meth-)acrylatmonomere. Ein entsprechendes Polymerisat umfasst dann ein entsprechendes Poly(meth)acrylat.

In der Patentliteratur ist eine Vielzahl von Verbindungen genannt (beispielsweise im Dokument DE 39 41 629 A1), die allesamt Diester der Acryl- oder Methacrylsäure sind und zur Herstellung eines Kunststoffs beziehungsweise einer Harzmatrix eines kunststoffbasierten Komposits geeignet sind, wie es in einem erfindungsgemäßen Fräsrohling vorliegt.

In einer bevorzugten Ausführung eines erfindungsgemäßen Fräsrohlings umfasst dieser Fräsrohling eine Harzmatrix, welche durch Polymerisation von ein oder mehreren Monomeren erzeugt wird, die ausgewählt sind aus der Gruppe bestehend Ethylenglykoldimethacrylat (EGDMA), 1,6-Hexandioldimethacrylat (HDDMA), Triethylenglykoldimethacrylat (TEGDMA), 1,10-Decandioldimethacrylat (DEDMA), 1,12-Dodecandioldimethacrylat (DODMA), ethoxyliertes Bisphenol-A-dimethacrylat, ethoxyliertes Bisphenol-A-dimethacrylat, wobei das Bisphenol mit 2 bis 4 mol Ethylenoxid umgesetzt wird und das Zwischenprodukt dann mit 2 mol Methacrylsäure abgesättigt wird, Polyethylenglykoldimethacrylat (PEGDMA), 7,7,9-Trimethyl-4,13-dioxo-3,14-dioxa-5,12-diazahexadecan-1,16-dioxydimethacrylat (UDMA), Butandioldimethacrylat, Tetraethylenglykoldimethacrylat, Neopentylglykoldimethacrylat sowie Bisphenol-A-Glycidyl-Methacrylat (Bis-GMA).

Im Einzelnen bevorzugt sind die entsprechenden Dimethacrylate bzw. Diacrylate des Dihydroxymethyltricyclo[5.2.1.0$^{2,6}$]decans, wie sie in den Druckschriften DE 1816823, DE 2419887, DE 2406557, DE 2931926, DE 3522005, DE 3522006, DE 3703120, DE 102005021332, DE 102005053775, DE 102006060983, DE 69935794 und DE 102007034457 beschrieben sind.

c) Initiatoren:

Bevorzugte erfindungsgemäße Fräsrohlinge sind durch Strahlenhärtung (photochemisch) und/oder durch chemische Härtung (Redoxreaktion) und/oder thermisch durch Aushärtung ein entsprechenden Zusammensetzung herstellbar, wobei die Zusammensetzung als Komponente a) ein anorganischen Füllstoff in einer Menge von wenigstens 70 Gew.- %, vorzugsweise wenigstens 80 Gew.-%, bezogen auf die Gesamtmaße des hergestellten Fräsrolings und/oder der eingesetzten Zusammensetzungen enthält oder eine füllstofffreie Zusammensetzung zur Herstellung eines Fräsrolings aus Kunststoff ist. Erfindungsgemäß bevorzugt zur Herstellung eines Fräsrolings ist die thermische Härtung einer entsprechenden Zusammensetzung, wobei die thermische Härtung beispielsweise durch Peroxidzerfall herbeigeführt wird.

Beispiele für geeignete Photosensibilisatoren sind alpha-Diketone, Benzoinalkylether, Thioxanthone, Benzophenone, Acylphosphinoxide, Acylgermanium-Verbindungen, Acetophenone, Ketale, Titanocene, sensibilisierende Farbstoffe, etc. Die Sensibilisatoren können alleine oder in Kombination angewendet werden. Konkrete Substanzbeispiele der unterschiedlichen Klassen finden sich beispielsweise in der DE 10 2006 019 092 A1, oder in der DE 39 41 629 C2.

Beispiele für Beschleuniger, die zusammen mit den Sensibilisatoren eingesetzt werden, sind tertiäre Amine, sekundäre Amine, Barbitursäuren, Zinnverbindungen, Aldehyde und Schwefelverbindungen. Konkrete Substanzbeispiele der unterschiedlichen Klassen finden sich in der DE 10 2006 019 092 oder in der DE 39 41 629 C2.

Weitere geeignete Initiatoren sowie Initiatorkombinationen sind in der DE 601 16 142 beschrieben.

Geeignete Photoinitiatoren sind dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm, gegebenenfalls in Kombination mit einem oder mehreren Coinitiatoren, die Aushärtung einer radikalisch härtbaren dentalen Zusammensetzung bewirken können.

Das Absorptionsmaximum von Campherchinon (CQ) liegt bei ca. 470 nm und somit im Bereich des blauen Lichts. Campherchinon (CQ) zählt zu den P12-Initiatoren und wird regelmäßig zusammen mit einem Coinitiator eingesetzt.

Ein geeignetes Katalysatorsystem enthält die Kombination eines alpha-Diketons und eines aromatischen tertiären Amins, bevorzugt ist die Kombination von Campherchinon (CQ) und Ethyl-*p-N,N*-dimethylaminobenzoat (DABE).

Ebenfalls bevorzugt ist die weitere Kombination des Systems "alpha-Diketon/aromatisches tertiäres Amin" mit einem Phosphinoxid, insbesondere mit dem Phenyl-bis(2,4,6-trimethylbenzoyl)phosphinoxid und/oder dem 2,4,6-Trimethylbenzoyldiphenylphosphinoxid. Bezüglich der Strukturen geeigneter Phosphinoxide wird auf die Druckschriften DE 38 01 511 C2, DE 10 2006 050 153 A1, EP 0 184 095 B1, DE 42 31 579 C2, EP 0 366 977 B1, US 7,081,485 B2, DE 32 36 026 A1, US 2007/0027229 A1, EP 0 262 629 B1, EP 0 073 413, US 7,148,382 B2, US 5,761,169, DE 197 08 294 A1, EP 0 057 474, EP 0 047 902 A, EP 0 007 508, DE 600 29 481 T2, EP 0 980 682 B1, EP 0 948 955 B1, EP 1 236 459 B1 und EP 0 173 567 A2 verwiesen.

Die in diesen Druckschriften angegebenen Phosphinoxide eignen sich besonders allein oder in Kombination mit dem System "alpha-Diketon/Amin" als Photopolymerisationsinitiatorsystem.

Weitere geeignete Photoinitiatoren sind in J.-P. Fouassier, Photoinitiation, Photopolymerization and Photocuring, Hanser Publishers, Munich, Vienna, New York 1995 sowie in J.F. Rabek (Hrsg.), Radiation Curing in Polymer Science and Technology, Vol. II, Elsevier Applied Science, London, New York 1993 beschrieben.

Dem Fachmann sind diverse Initiatoren für eine chemische Aushärtung bekannt. Es sei insoweit exemplarisch auf die EP 1 720 506 verwiesen. Initiatoren zur chemischen Aushärtung werden auch in den oben bereits erwähnten Druckschriften DE 10 2006 019 092 sowie in der DE 39 41 629 beschrieben.

Bevorzugte Initiatoren zur chemischen Härtung sind Dibenzoylperoxid, Dilauroylperoxid insbesondere Dibenzoylperoxid in Kombination mit Aminen wie N,N-Dimethyl-p-toluidin, N,N-Dihydroxyethyl-p-toluidin sowie strukturverwandten Aminen.

Dualhärtende Systeme umfassen eine Kombination aus Photoinitiatoren und Initiatoren zur chemischen Aushärtung.

Neben den oxidativ wirksamen organischen Peroxidverbindungen können als Redoxsysteme auch Barbitursäuren bzw. Barbitursäurederivate sowie Malonylsulfamide verwendet werden.

Von den Barbitursäuresystemen sind die sogenannten "Bredereck-Systeme" von hoher Bedeutung. Beispiele geeigneter "Bredereck-Systeme" sowie Verweise auf die entsprechende Patentliteratur findet man in der EP 1 839 640 sowie in DE 1495520, WO 02/092021 oder in WO 02/092023.

Anstelle der Barbitursäuren können auch deren Salze verwendet werden. Beispiele hierzu finden sich in den folgenden Dokumenten: EP 1 872 767, EP 2 070 506, EP 1 881 010, DE 10 2007 050 763, US 6,288,138, DE 11 2006 001 049, US 7,214,726 sowie EP 2 070 935.

Geeignete Malonylsulfamide sind in der EP 0 059 451 beschrieben. Bevorzugte Verbindungen sind dabei 2,6-Dimethyl-4-isobutylmalonylsulfamid, 2,6-Diisobutyl-4-propylmalonylsulfamid, 2,6-Dibutyl-4-propylmalonylsulfamid, 2,6-Dimethyl-4-ethylmalonylsulfamid sowie 2,6-Diocytyl-4-isobutylmalonylsulfamid.

Ferner können Schwefelverbindungen in der Oxidationsstufe +2 oder +4 wie Natriumbenzolsulfinat oder Natriumparatoluolsulfinat eingesetzt werden.

Zur Beschleunigung der Aushärtung kann die Polymerisation in Gegenwart von Schwermetallverbindungen wie Ce, Fe, Cu, Mn, Co, Sn oder Zn durchgeführt werden, wobei Kupferverbindungen besonders bevorzugt sind. Die Schwermetallverbindungen werden bevorzugt in Form löslicher organischer Verbindungen eingesetzt. Bevorzugte Kupferverbindungen sind dabei Kupferbenzoat, Kupferacetat, Kupferethylhexanoat, Kupferdi(methacrylat), Kupferacetylacetonat und Kupfernaphthenat.

Werden die Peroxide erwärmt, so zerfallen sie und bilden freie Radikale, die befähigt sind, die Polymerisation zu starten. Das am weitesten verbreitete System zur thermischen Polymerisation ist die Verwendung von Dibenzoylperoxid. Weitere thermische Initiatoren sind Ketonperoxide, Peroxyketale, Hydroperoxide, Dialkylperoxide, Diacylperoxide, Peroxyester und Peroxydicarbonate wie Dicumylperoxid, Chlorbenzoylperoxid, t-Butylperbenzoat, Dilauroylperoxid, Cumolhydroperoxid, 3,5,5-Trimethylhexansäure-tert.-butylperoxyester sowie Azoverbindungen wie 2,2-Azobisisobutyronitril, 2,2'-Azobis-2,4-dimethylvaleronitril, 2,2'-Azobis-1-cyclohexancabonitril oder Dimethyl-2-2'-azobisisobutyrat. Auch Substanzen wie Natrium- oder Kaliumpersulfat zerfallen thermisch und sind in diesem Zusammenhang geeignete Verbindungen. Diese Substanzen können einzeln oder in Mischungen untereinander verwendet werden. Hierzu müssen die radikalisch härtbaren dentalen Zusammensetzungen lediglich auf die vom Hersteller angegebene Zerfallstemperatur der jeweiligen Peroxide erwärmt werden. Vorteilhafterweise werden die radikalisch härtbaren Zusammensetzungen auf eine Temperatur oberhalb der Zerfallstemperatur erwärmt und dort für einige Zeit belassen, damit das Polymerisat Zeit zur Relaxation hat. Der Fachmann findet die optimale Temperatur dadurch, dass er die Temperatur zur Härtung sukzessive bis zu dem Punkt erhöht, an dem das Polymerisat keine wesentlichen Verbesserungen an den an ihm gemessenen wichtigen Parameter wie Biegefestigkeit, E-Modul und Wasseraufnahme aufweist.

Vorzugsweise wird die thermische Härtung so ausgeführt, dass die radikalisch härtbare Zusammensetzung in eine Blockform überführt wird, wo sie bei Temperaturen von 80°C bis 150°C und bei einem Druck von 100 bis 300 bar ausgehärtet wird.

d) Additive:

Ein erfindungsgemäßer Fräsrohling umfasst in manchen Fällen ein oder mehrere weitere(s) Additiv(e).

Diese Additive können verschiedene Funktionen haben. Übliche Additive für den Einsatz in dentalen Werkstoffen sind dem Fachmann bekannt, je nach gewünschter Funktion wird er das oder die geeigneten Additive auswählen. Beispielhaft sind im Folgenden typische Additive und ihre Funktionen beschrieben.

UV-Absorber, die beispielsweise durch ihre konjugierten Doppelbindungssysteme und aromatischen Ringe befähigt sind, UV Strahlung zu absorbieren, sind in manchen Fällen Bestandteil eines erfindungsgemäßen Fräsrohlings. Beispiele von UV-Absorbern sind 2-Hydroxy-4-methoxybenzophenon, Salicylsäurephenylester 3-(2'-Hydroxy-5'-methylphenyl)benzotriazol und Diethyl-2,5-dihydroxyterephthalat. Die Polymerisate enthalten diese Additive, um ihre Farbstabilität zu gewährleisten.

Da indirekte dentale Restaurationen dazu vorgesehen sind, Zähne möglichst naturgetreu zu restaurieren, ist es erforderlich, die erfindungsgemäßen Fräsrohlinge in unterschiedlichen Farbtönen bereitzustellen. Zu diesem Zweck enthalten erfindungsgemäße Fräsrohlinge in der Regel anorganische Farbstoffe und/oder organische Pigmente, vorzugsweise in sehr geringen, jedoch für die genannten Zwecke ausreichenden Mengen.

Weitere optionale Additive sind dentale Arzneimittel, Mikrobizide, vorzugsweise Bakterizide oder Fluoreszenzmittel, die ebenfalls eingesetzt werden, um das natürliche Erscheinungsbild von Zähnen abzubilden.

Ein erfindungsgemäßer Fräsrohling umfasst einen ungewöhnlich hohen Anteil an Wasser, der üblicherweise durch eine entsprechende Vorkonditionierung in den Fräsrohling eingebracht wird. Dieser Anteil an Wasser soll bei der Lagerung eines erfindungsgemäßen Fräsrohlings vorzugsweise nicht signifikant geringer werden, so dass es bevorzugt ist, Maßnahmen zu treffen, welche den Fräsrohling davor bewahren, in unerwünschter Weise Wasser zu verlieren. Insoweit stehen dem Fachmann eine Reihe von Schutzmaßnahmen zur Verfügung, die von der Lagerung in Kontakt mit Wasser, der Oberflächenversiegelung eines erfindungsgemäßen Fräsrohlings z. B. durch Infiltration und Aushärtung von Monomeren bis hin zum wasserdichten Umschließen des Fräsrohlings reichen.

Bevorzugt ist ein erfindungsgemäßer Fräsrohling (wie vorstehend definiert, vorzugsweise wie vorstehend als bevorzugt oder besonders bevorzugt bezeichnet), wobei der Fräsrohling wasserdicht versiegelt oder umschlossen ist, vorzugsweise wasserdicht umschlossen ist von einem wasserdichten Behältnis, vorzugsweise einem Blister, und/oder einem wasserdichten Lack und/oder einer wasserdichten Hülle aus Wachs.

Der Begriff "wasserdicht" bedeutet für die Zwecke des vorliegenden Textes, dass ein erfindungsgemäßer Fräsrohling, der "wasserdicht" versiegelt oder umschlossen ist, bei einer Lagertemperatur von 25 °C und einem Umgebungsdruck von 1013 hPa bei einer relativen Luftfeuchte von 30 % auch nach einer Lagerung von einem Jahr noch immer Wasser in einer Menge von wenigstens 25 % der Wasseraufnahme $W_{SP}$ umfasst, also noch immer erfindungsgemäß ist.

Vorzugsweise ist ein bevorzugter erfindungsgemäßer Fräsrohling (mit einem entsprechend hohen Wassergehalt) sogar derart wasserdicht versiegelt oder umschlossen, dass er unter den angegebenen Lagerbedingungen auch nach einem Jahr noch Wasser in einer Menge von wenigstens 50 %, bevorzugt wenigstens 75 %, besonders bevorzugt wenigstens 90 % der Wasseraufnahme $W_{SP}$ umfasst.

Die vorliegende Erfindung betrifft auch die Verwendung eines erfindungsgemäßen Fräsrohlings zur Herstellung eines Formkörpers zur Verwendung als indirekte dentale Restauration, vorzugsweise eines Formkörpers ausgewählt aus der Gruppe bestehend aus Inlay, Onlay, Teilkrone, Krone, Veneer (Verblendung) und Brücke. Wie bereits weiter oben ausgeführt, wird ein Formkörper vorzugsweise mittels Fräsen aus einem erfindungsgemäßen Fräsrohling hergestellt.

Die vorliegende Erfindung betrifft auch ein Verfahren zur Herstelllung eines erfindungsgemäßen Fräsrohlings oder eines daraus gefertigten Formkörpers zur Verwendung als indirekte dentale Restauration, mit folgenden Schritten:

(i) Herstellen oder Bereitstellen eines Fräsrohlings umfassend Wasser in einer Menge von weniger als 25 % der Wasseraufnahme $W_{sp}$, bevorzugt von weniger als 15 %, besonders bevorzugt von weniger als 10 %,

(ii) Einstellen von Bedingungen, bei denen der in Schritt (i) bereitgestellte oder hergestellte Fräsrohling Wasser aufnimmt und Beibehalten dieser Bedingungen, bis der Fräsrohling Wasser in einer Menge von wenigstens 25 % der Wasseraufnahme $W_{sp}$ umfasst, sowie vorzugsweise wasserdichtes Versiegeln oder Umschließen des erzeugten Fräsrohlings.

Ein erfindungsgemäßes Verfahren zur Herstellung eines Formkörpers zur Verwendung als indirekte dentale Restauration umfasst vorzugsweise den zusätzlichen Schritt:
(iii) Fräsen des Formkörpers aus dem in Schritt (ii) erzeugten Fräsrohling, vorzugsweise mittels einer CAD/CAM-Fräseinrichtung, sowie vorzugsweise Polieren des Formkörpers.

Ein erfindungsgemäßes Verfahren zur Herstellung eines erfindungsgemäßen Fräsrohlings umfasst vorzugsweise einen zusätzlichen Schritt, wobei der Fräsrohling wasserdicht versiegelt oder wasserdicht umschlossen wird, vorzugsweise wasserdicht umschlossen wird von einem wasserdichten Behältnis, vorzugsweise einem wasserdichten Blister, und/oder einem wasserdichten Lack und/oder einer wasserdichten Hülle aus Wachs. Die obigen Ausführungen zum Begriff "wasserdicht" und entsprechenden bevorzugten Ausgestaltungen gelten entsprechend.

Nachdem aus einem erfindungsgemäßen Fräsrohling eine indirekte dentale Restauration, beispielsweise eine Krone, im CAD/CAM - Verfahren gefräst worden ist und ein Zahnstumpf präpariert wurde, wird ein Zahnarzt üblicherweise die innere Oberfläche der präparierten indirekten dentalen Restauration, beispielsweise der Krone, mittels Sandstrahlen aufrauen, dann säubern und primen. Er wird dann das Bonding auf den Stumpf auftragen und härten und schließlich einen Befestigungszement auf die indirekte dentale Restauration applizieren (beispielsweise in die Krone einfüllen) und die indirekte dentale Restauration dann am Zahnstumpf ansetzen (beispielsweise die Krone auf den Zahnstumpf aufsetzen).

Beschrieben wird auch die Anwendung des Verfahrens der Wasseraufnahme zum Einstellen des Grads der Quellung eines Fräsrohlings vor dem Fräsen zur Herstellung eines dentales Formkörpers zur Verwendung als indirekte dentale Restauration.

Es ist aus dem Stand der Technik nicht bekannt, dass Fräsrohlinge gezielt durch Wasseraufnahme vor dem Fräsen auf einen definierten Quellgrad (und eine entsprechende Fähigkeit zur weiteren Quellung) eingestellt werden, um auf diesem Wege durch das Fräsen zu entsprechenden dentalen Formkörpern zu gelangen, die besonders gut als indirekte dentale Restauration eingesetzt werden können. Es ist aus dem Stand der Technik nicht bekannt, dass eine solche Maßnahme vorteilhafterweise dazu führt, dass die resultierenden dentalen Formkörper eine besonders hohe Retention besitzen. Hinsichtlich bevorzugter Ausgestaltungen der beschriebenen Anwendung sei auf sämtliche vorstehenden Ausführungen verwiesen, die entsprechend gelten, mutatis mutandis.

Die vorliegende Erfindung betrifft auch einen Kit zur Herstellung von indirekten dentalen Restaurationen, umfassend:

- zwei oder mehr Fräsrohlinge in unterschiedlichen Farben
  sowie

- ein oder mehrere dentale Zusammensetzungen zur Befestigung eines aus einem der Fräsrohlinge gefrästen Formkörpers an einem dentalen Objekt in der Mundhöhle, vorzugsweise ausgewählt aus der Gruppe bestehend aus Primern, Adhäsiven und Befestigungszementen,
  sowie vorzugsweise

- weiteres Zubehör wie Pinsel, Poliermittel und Mischkanülen.

Die vorliegende Erfindung betrifft auch ein therapeutisches oder kosmetisches Verfahren zur indirekten dentalen Restauration, mit folgenden Schritten:

i) Herstellen oder Bereitstellen eines vorzugsweise erfindungsgemäßen Fräsrohlings (wie vorstehend und in den Anspruch definiert, vorzugsweise wie vorstehend als bevorzugt bezeichnet) umfassend Wasser in einer Menge von weniger als 25 % der Wasseraufnahme $W_{SP}$, bevorzugt von weniger als 15 %, besonders bevorzugt von weniger als 10 %,

ii) Einstellen von Bedingungen, bei denen der in Schritt (i) bereitgestellte oder hergestellte Fräsrohling Wasser aufnimmt und Beibehalten dieser Bedingungen, bis der Fräsrohling Wasser in einer Menge von wenigstens 25 %, vorzugsweise wenigstens 50 %, bevorzugt wenigstens 75 %, besonders bevorzugt wenigstens 90 % der Wasseraufnahme $W_{SP}$ umfasst, sowie gegebenenfalls wasserdichtes Versiegeln oder Umschließen des erzeugten (vorzugsweise erfindungsgemäßen) Fräsrohlings,

wobei die Bedingungen vorzugsweise beibehalten werden, bis die Differenz zwischen der Wasseraufnahme $W_{SP}$ und dem Wassergehalt des Fräsrohlings kleiner ist als 10 $\mu$g/mm$^3$, vorzugsweise kleiner ist als 8 $\mu$g/mm$^3$, bevorzugt kleiner ist als 4 $\mu$g/mm$^3$,

iii) Fräsen eines Formkörpers zur Verwendung als indirekte dentale Restauration aus dem in Schritt (ii) erzeugten Fräsrohling sowie gegebenenfalls Polieren des Formkörpers,

wobei das Fräsen auf Basis dreidimensionaler Geometriedaten der Restauration mittels einer CAD/CAM-Fräseinrichtung erfolgt,

(iv) Befestigen des in Schritt (iii) hergestellten Formkörpers an einem dentalen Objekt in der Mundhöhle.

Verweise auf Dokumente:

[1] Roggendorf MJ, Kunzi B, Ebert J, Roggendorf HC, Frankenberger R, Reich SM; Seven-year clinical performance of CEREC-2 all-ceramic CAD/CAM restorations placed within deeply destroyed teeth. Clin Oral Investig 2012, 16(5), 1413-1424.

[2] Frankenberger R, Petschelt A, Krämer N; Leucite-reinforced glass ceramic inlays and onlays after six years: clinical behavior. Oper Dent 2000, 25(6), 459-465.

[3] Frankenberger R, Taschner M, Garcia-Godoy F, Petschelt A, Krämer N; Leucite-reinforced glass ceramic inlays and onlays after 12 years. Adhes Dent 2008, 10(5), 393-398.

[4] Otto T, De Nisco S; Computer-aided direct ceramic restorations: a 10-year prospective clinical study of Cerec CAD/CAM inlays and onlays. Int J Prosthodont 2002, 15(2), 122-128.

[5] Posselt A, Kerschbaum T; Longevity of 2328 chairside Cerec inlays and onlays. Int J Comput Dent 2003, 6(3), 231-248.

[6] Otto T, Schneider D; Long-term clinical results of chairside Cerec CAD/CAM inlays and onlays: a case series. Int J Prosthodont 2008, 21(1), 53-59.

[7] Reiss B; Clinical results of CEREC inlays in a dental practice over a period of 18 years. Int J Comput Dent 2006, 9(1), 11-22.

[8] Kassem AS, Atta O, El-Mowafy O; Combined effects of thermocycling and loadcycling on microleakage of computer-aided design/computer-assisted manufacture molar crowns. Int J Prosthodont 2011, 24(4), 376-378.

[9] Kassem AS, Atta O, El-Mowafy O; Fatigue resistance and microleakage of CAD/CAM ceramic and composite molar crowns. J Prosthodont 2012, 21(1), 28-32.

[10] Attia A, Abdelaziz KM, Freitag S, Kern M; Fracture load of composite resin and feldspathic all-ceramic CAD/CAM crowns. J Prosthet Dent 2006, 95(2), 117-123.

[11] Ramirez-Sebastia A, Bortolotto T, Roig M, Krejci I; Composite vs Ceramic Computer-aided Design/Computer-assisted Manufacturing Crowns in Endodontically Treated Teeth: Analysis of Marginal Adaptation. Oper Dent 2013, 38(6), 663-673.

[12] Lauvahutanon S, Takahashi H, Shiozawa M, Iwasaki N, Asakawa Y, Oki M, Finger WJ, Arksornnukit M; Mechanical properties of composite resin blocks for CAD/CAM. Dent Mater J 2014, 33(5), 705-710.

[13] Heintze SD; Crown pull-off test (crown retention test) to evaluate the bonding effectiveness of luting agents. Dental Materials 2010, 36(3), 193-206.

[14] Bähr N, Keul C, Edelhoff D, Eichberger M, Roos M, Gernet W, Stawarczyk B; Effect of different adhesives combined with two resin composite cements on shear bond strength to polymeric CAD/CAM materials. Dent Mater J 2013, 32(3), 492-501.

[15] Güngör MB, Nemli SK, Bal BT, Ünver S, Dogan A; Effect of surface treatments on shear bond strength of resin composite bonded to CAD/CAM resin-ceramic hybrid materials. Adv Prosthodont 2016, 8, 259-266.

[16] Elsaka SE; Bond strength of novel CAD/CAM restorative materials to self-adhesive resin cement: the effect of surface treatments. Adhes Dent 2014, 16(6), 531-540.

[17] Yoshida K, Kamada K, Atsuta M; Effects of two silane coupling agents, a bonding agent, and thermal cycling on the bond strength of a CAD/CAM composite material cemented with two resin luting agents. J Prosthet Dent 2001, 85(2), 184-189.

[18] Keul C, Müller-Hahl M , Eichberger M , Liebermann A , Roos M , Edelhoff D , Stawarczyk B; Impact of different adhesives on work of adhesion between CAD/CAM polymers and resin composite cements. J Dent 2014, 42(9), 1105-1114.

[19] Stawarczyk B, Basler T, Ender A, Roos M, Ozcan M, Hammerle C; Effect of surface conditioning with airborne-particle abrasion on the tensile strength of polymeric CAD/CAM crowns luted with self-adhesive and conventional resin cements. J Prosthet Dent 2012, 107(2), 94-101.

[20] Gilbert S; Einfluss der Testmethode auf den Verbund zwischen CAD/CAM-Hochleistungspolymer und kunststoffbasierten Befestigungsmaterialien nach unterschiedlichen Vorbehandlungen. Dissertation, Ludwig-Maximilians-Universität München 2014.

[21] Martin A; Zugverbundfestigkeitsuntersuchungen von Hochleistungskunststoffen nach unterschiedlichen Vorbehandlungsmethoden. Dissertation, Ludwig-Maximilians-Universität München 2015.

[22] Stawarczyk B, Stich N, Eichberger M, Edelhoff D, Roos M, Gernet W, Keul C; Long-term tensile bond strength of differently cemented nanocomposite CAD/CAM crowns on dentin abutment. Dental Materials 2014, 30(3), 334-342.

[23] Basler T; Effect of surface conditioning with air-abrasion on the tensile strength of polymeric CAD/CAM crowns luted with self-adhesive and conventional resin cements. Dissertation, Universität Zürich 2011

[24] Frankenberger R, Hartmann VE, Krech M, Krämer N, Reich S, Braun A, Roggendorf M; Adhäsive Befestigung neuer CAD/CAM-Materialien. Int J Comput Dent 2015, 18(1), 9-20.

[25] Shetty R, Shenoy K, Dandekeri S, Suhaim KS, Ragher M, Francis J; Resin-Matrix Ceramics - An Overview. International Journal of Recent Scientific Research 2015, 6(11), 7414-7417.

[26] Vanoorbeek S, Vandamme K, Lijnen I, Naert I; Computer-aided designed/computer-assisted manufactured composite resin versus ceramic single-tooth restorations: a 3-year clinical study. Int J Prosthodont. 2010, 23(3), 223-230.

[27] Schepke U, Meijer HJA, Vermeulen KM, Raghoebar GM, Cune MS; Clinical Bonding of Resin Nano Ceramic Restorations to Zirconia Abutments: A Case Series within a Randomized Clinical Trial. Clin Implant Dent Relat Res 2016, 18(5), 984-992.

Das Dokument US 2014/0200284 A1 offenbart einen Fräsrohling ("mill blank"), der aus einer näher definierten dentalen Zusammensetzung hergestellt ist.

Beispiele:

Abkürzungen:

Bis-EMA2,6: Ethoxyliertes Bisphenol A-Dimethacrylat mit durchschnittlich 2,6 Ethylenoxideinheiten

Bis-EMA4: Ethoxyliertes Bisphenol A-Dimethacrylat mit durchschnittlich 4 Ethylenoxideinheiten

Bis-EMA6: Ethoxyliertes Bisphenol A-Dimethacrylat mit durchschnittlich 6 Ethylenoxideinheiten

Bis-EMA10: Ethoxyliertes Bisphenol A-Dimethacrylat mit durchschnittlich 10 Ethylenoxideinheiten

TCDDMA: Bis(methacryloyloxymethyl)tricyclo[$5.2.1.0^{2,6}$]decan

UDMA: 7,7,9-Trimethyl-4,13-dioxo-3,14-dioxa-5,12-diazahexadecan-1,16-dioxydimethacrylat

TEGDMA: Triethylenglycoldimethacrylat

HDDMA: 1,6-Hexandioldimethacrylat

DODMA: 1,12-Dodecandioldimethacrylat

Dentalglas 1: Barium-Aluminium-Borosilikat-Glas (D50 0,8 $\mu$m / D25 0,5 $\mu$m / D75 1,0 $\mu$m), silanisiert

Dentalglas 2: Barium-Aluminium-Borosilikat-Glas (D50 2,7 $\mu$m / D25 1,4 $\mu$m / D75 6,1 $\mu$m) , silanisiert

Dentalglas 3: Barium-Aluminium-Borosilikat-Glas (D50 1,1 $\mu$m / D25 0,7 $\mu$m / D75 1,4 $\mu$m) , silanisiert

Dentalglas 4: Barium-Aluminium-Borosilikat-Glas (D50 1,4 $\mu$m / D25 1,1 $\mu$m / D75 2,1 $\mu$m) , silanisiert

Dentalglas 5: Barium-Aluminium-Borosilikat-Glas (D50 1,4 $\mu$m / D25 0,8 $\mu$m / D75 2,9 $\mu$m) , silanisiert

Nano-$SiO_2$: nicht agglomerierte, nicht aggregierte Kieselsäure (D50 40 nm) , silanisiert

BPO: Dibenzoylperoxid

Herstellung der Komposit-Pasten:
Die einzelnen Komponenten wurden entsprechend der in den Tabellen 2 bis 14 angegebenen Anteile eingewogen, für 30 Minuten bei 50 U/min an einem Laborkneter (PC Laborsystem, Magden CH) homogenisiert und anschließend am Laborkneter für 15 Minuten bei 50 U/min und -0,85 bar entlüftet.

Herstellung der Komposit-Blöcke:
Zur Herstellung der Komposit-Blöcke wurden die einzelnen Pasten in Formen (15 mm x 15 mm x 20 mm) gefüllt. Die Aushärtung erfolgte isostatisch bei 250 bar und folgendem Temperaturprogramm (20 °C - 2 °C/min - 120 °C (30 min) - 5 °C/min - 20 °C)

Biaxiale Biegefestigkeit (BBF): Die Biaxiale Biegefestigkeit wurde analog DIN EN ISO 6872:2009 (7.3.3) bestimmt. Dazu wurden aus den Komposit-Blöcken zunächst in einer 5-Achs Fräsmaschine (250i, imes-icore GmbH) Zylinder mit einem Durchmesser von 14 mm geschliffen. Aus diesen Zylindern wurden dann mit einer Hochgeschwindigkeitssäge (IsoMet 4000, Buehler) Scheiben mit einer Dicke von 1,2 mm hergestellt, entgratet, geschliffen und poliert. Die Proben wurden mit einer Traversengeschwindigkeit von 1 mm/min bis zum Bruch belastet und die biaxiale Biegefestigkeit entsprechend der unter 7.3.3.4 angegebenen Formel berechnet. Als Poisson'sche Querkontraktionszahl wurde ein Wert von 0,25 eingesetzt.

3-Punkt-Biegefestigkeit (3PBF): Die Biegefestigkeit wurde analog DIN EN ISO 6872:2009 (7.3.2) bei einer Stützweite von 12 mm und einem Auflagerollendurchmesser von 2 mm bestimmt. Dazu wurden aus den Komposit-Blöcken Probekörper mit einer Breite von 4 mm, einer Dicke von 1,2 mm und einer Länge von 18 mm mit einer Hochgeschwindigkeitssäge (IsoMet 4000, Buehler) hergestellt, entgratet, geschliffen und poliert. Die Proben wurden mit einer Traversengeschwindigkeit von 1 mm/min bis zum Bruch belastet und die 3-Punkt-Biegefestigkeit entsprechend der unter 7.3.2.4.1 angegebenen Formel berechnet.

Elastizitätsmodul (E): Der Elastizitätsmodul wurde analog der Berechnung in ADA Specification No. 27 - 1993 (7.8.4.2) als Steigung der Spannung-Dehnungs-Kurve der 3-Punkt-Biegefestigkeitsmessung (siehe oben) im linear-elastischen Bereich ermittelt.

$$E = \frac{3\,L}{4\,b\,h^3}\,\frac{\Delta F}{\Delta d}$$

L: Stützweite

b: Probenbreite

h: Probendicke

$\Delta d$: Deformation im linear-elastischen Bereich

$\Delta F$: Kraftänderung bei einer Deformation $\Delta d$

Wasseraufnahme ($W_{SP}$): Die Wasseraufnahme wurde analog EN ISO 4049:2009 (D)(7.12) bestimmt; nachfolgend werden die Bestimmungsmethode zusammengefasst und Abweichungen (insbesondere hinsichtlich der Probengeometrie) von der Vorgehensweise gemäß EN ISO 4049:2009 (D) angegeben:
Aus den hergestellten Komposit-Blöcken wurden Probekörper mit einer Länge von 14,7 mm, einer Breite von 14,7 mm und einer Dicke von 0,5 mm mit einer Hochgeschwindigkeitssäge (IsoMet 4000, Buehler) hergestellt, entgratet, geschliffen und poliert. Die Probekörper wurden (analog 7.12.3.1) im Exsikkator bei 37 °C bis zur Massenkonstanz getrocknet und die Masse ($m_1$) auf 0,1 mg, sowie (analog 7.12.3.2) die Länge, die Breite und die Dicke auf 0,01 mm genau bestimmt; hieraus wurde das Volumen V in Kubikmillimeter berechnet. Anschließend wurden die Probekörper (analog 7.12.3.3) für 7 Tage bei 37 °C in Wasser gelagert. Nach 7 Tagen wurden die Probekörper herausgenommen, mit Wasser abgespült, abgetupft, für 15 Sekunden in der Luft hin- und hergeschwenkt und 1 Minute nach dem Herausnehmen aus dem Wasser auf 0,1 mg genau gewogen ($m_2$). Nach dieser Wägung wurden (analog 7.12.3.4) die Probekörper im Exsikkator bei 37 °C bis zur Massenkonstanz getrocknet und die Masse ($m_3$) auf 0,1 mg bestimmt. Die Wasseraufnahme wurde entsprechend der unter 7.12.4.1 angegebenen Gleichung (2) berechnet.

Wasserbehandlung/-konditionierung der Kompositblöcke und Bestimmung des Konditionierungsgrades: Hergestellte Kompositblöcke wurden im Exsikkator bei 37 °C bis zur Massenkonstanz getrocknet und die Masse der so getrockneten Kompositblöcke ($m_0$) auf 0,1 mg, sowie die Länge, die Breite und die Dicke auf 0,01 mm genau bestimmt; hieraus wurde das Volumen V in Kubikmillimeter berechnet. Anschließend wurden die Kompositblöcke für 1 Woche bei 60 °C in Wasser gelagert. Nach 1 Woche wurden die Kompositblöcke herausgenommen, mit Wasser abgespült, abgetupft, für 15 Sekunden in der Luft hin- und hergeschwenkt und 1 Minute nach dem Herausnehmen aus dem Wasser auf 0,1 mg genau gewogen ($m_{1\,Woche}$). Anschließend wurden die Kompositblöcke erneut bei 60 °C in Wasser gelagert. In wöchentlichen Intervallen wurden die Kompositblöcke erneut herausgenommen, mit Wasser abgespült, abgetupft, für 15 Sekunden in der Luft hin- und hergeschwenkt und 1 Minute nach dem Herausnehmen aus dem Wasser auf 0,1 mg genau gewogen ($m_{2\,Wochen}$, $m_{3\,Wochen}$, usw.). Der Konditionierungsgrad wurde für die jeweiligen Termine jeweils im Verhältnis zu der an 0,5 mm dicken Scheiben gleicher Zusammensetzung bestimmten Wasseraufnahme ($W_{SP}$) (siehe oben) nach folgender Formel berechnet.

$$Konditionierungsgrad_{n\,Wochen}[\%] = \frac{m_{n\,Wochen} - m_0}{V_{Kompositblock} \times W_{SP}} \times 100\%$$

Analog wurde der Konditionierungsgrad auch bei einer Lagertemperatur von 37 °C bestimmt. (Anmerkung: Die Kompositblöcke erreichen aufgrund ihrer Dimensionen ihre vollständige Wassersättigung langsamer als die für die Bestimmung der Wasseraufnahme verwendeten 0,5 mm dicken Scheiben.)

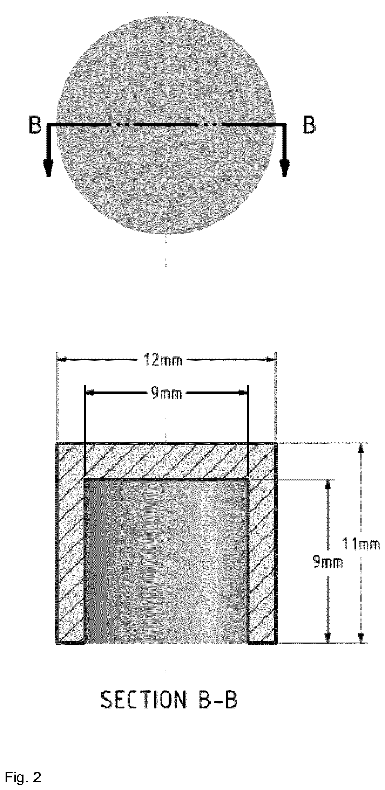

Lineare Quellung (LQ): In einer 5-Achs Fräsmaschine (250i, imes-icore GmbH) wurden idealisierte Kronen als Probekörper aus den Kompositblöcken hergestellt. Diese idealisierten Kronen sind einseitig geschlossene Hohlzylinder (vgl. Figur 2).

Die Höhe beträgt 11 mm, der Außendurchmesser 12 mm und der Innendurchmesser 9 mm. Dies entspricht einer Wandstärke von 1,5 mm. Die Stärke der Deckplatte beträgt 2 mm. Anschließend wurden die idealisierten Kronen entgratet, geschliffen und poliert. Die resultierenden Probekörper wurden im Exsikkator bei 37 °C bis zur Massenkonstanz getrocknet und der Innendurchmesser der getrockneten Probekörper an der Zylinderbasis an zwei Stellen orthogonal zueinander auf 0,001 mm genau bestimmt ($L_1$ und $L_2$). Anschließend wurden die Probekörper für 7 Tage (1 Woche) bei 37 °C in Wasser gelagert. Nach 7 Tagen wurden die Probekörper herausgenommen, mit Wasser abgespült, abgetupft und für 15 Sekunden in der Luft hin- und hergeschwenkt; 1 Minute nach dem Herausnehmen aus dem Wasser wurde der Innendurchmesser an der Zylinderbasis an den gleichen zwei Stellen wie zuvor auf 0,001 mm genau bestimmt ($L_3$ und $L_4$). Nach der Messung wurden die Probekörper für weitere 7 Tage (1 Woche) bei 37 °C in Wasser gelagert (gesamt: 2 Wochen). Danach wurden die Probekörper herausgenommen, wie oben beschrieben getrocknet und erneut der Innendurchmesser an den gleichen Stellen auf 0,001 mm genau bestimmt ($L_5$ und $L_6$). Nach der Messung wurden die Probekörper für weitere 14 Tage (2 Wochen) bei 37 °C in Wasser gelagert (gesamt: 4 Wochen). Danach wurden die Probekörper herausgenommen, wie oben beschrieben getrocknet und erneut der Innendurchmesser an den gleichen Stellen auf 0,001 mm genau bestimmt ($L_7$ und $L_8$). Nach der Messung wurden die Probekörper für weitere 28 Tage (4 Wochen) bei 37 °C in Wasser gelagert (gesamt: 8 Wochen). Danach wurden die Probekörper herausgenommen, wie oben beschrieben getrocknet und erneut der Innendurchmesser an den gleichen Stellen auf 0,001 mm genau bestimmt ($L_9$ und $L_{10}$). Die lineare Quellung in % zu den jeweiligen Messterminen ergibt sich entsprechend der folgenden Formeln.

$$LQ_{1Woche} \, [\%] = \frac{\frac{L_3 + L_4}{2} - \frac{L_1 + L_2}{2}}{\frac{L_1 + L_2}{2}} \times 100\%$$

$$LQ_{2Wochen} \, [\%] = \frac{\frac{L_5 + L_6}{2} - \frac{L_1 + L_2}{2}}{\frac{L_1 + L_2}{2}} \times 100\%$$

$$LQ_{4Wochen} \, [\%] = \frac{\frac{L_7 + L_8}{2} - \frac{L_1 + L_2}{2}}{\frac{L_1 + L_2}{2}} \times 100\%$$

$$LQ_{8Wochen} \, [\%] = \frac{\frac{L_9 + L_{10}}{2} - \frac{L_1 + L_2}{2}}{\frac{L_1 + L_2}{2}} \times 100\%$$

Glührückstand: Zur Bestimmung des Glührückstandes wurden Tiegel für 10 Stunden auf 150 °C erwärmt, im Exsikkator auf Raumtemperatur abkühlen gelassen und anschließend auf 0,1 mg genau gewogen ($m_1$). Es wurden ca. 1 g des jeweiligen Komposit-Blockes zerkleinert, gemörsert und auf 0,1 mg genau im Tiegel eingewogen ($m_2$). Es wurde für 3 Stunden im Muffelofen auf 575 °C erhitzt, danach wurden die Tiegel im Exsikkator auf Raumtemperatur abkühlen gelassen und anschließend wurde die Masse (Tiegel mit Rückstand) auf 0,1 mg genau bestimmt ($m_3$). Der Glührückstand wurde nach folgender Formel berechnet.

$$Glührückstand \, [\%] = \frac{m_3 - m_1}{m_2} \times 100\%$$

Haftung: Als idealisierte Stümpfe wurden Zirkondioxid-Abutments hergestellt. Die Zirkondioxid-Abutments wurden als Kegelstümpfe gestaltet, die einen Durchmesser von 10,0 mm an der Stirnfläche, einen Winkel von 5° und eine Höhe von 15 mm aufwiesen. In einer 5-Achs Fräsmaschine (250i, imes-icore GmbH) wurden idealisierte Kronen in Form von Käppchen als Probekörper aus den Kompositblöcken (zum Konditionierungsgrad siehe unten) hergestellt. Die Innenfläche der Kronen entsprach der Außenfläche der Zirkondioxid-Abutments, wobei ein Spalt von 70 $\mu$m (zur Aufnahme von Befestigungsmitteln) mit einberechnet wurde. Der Innendurchmesser an der Stirnfläche betrug dementsprechend 10,14 mm. Der Winkel betrug entsprechend ebenfalls 5° und die Innenhöhe betrug 6,55 mm. Die Wandstärke betrug überall 1,5 mm. Als Vergleich wurden Keramikkronen (IPS e.max CAD, Ivoclar Vivadent) mit den gleichen Abmessungen hergestellt.

Die Kroneninnenfläche wurde mit Aluminiumdioxid (50 $\mu$m) sandgestrahlt (1,5 bar). Anschließend wurde Haftvermittler (Ceramic Bond, VOCO GmbH) auf Zirkondioxid-Abutment und Kroneninnenfläche aufgetragen und für 60 Sekunden trocknen gelassen. Anschließend wurden die Kronen mit einem Befestigungssystem auf Kompositbasis (Bifix QM, VOCO GmbH) auf den Abutments befestigt. Die Aushärtung erfolgte für 24 Stunden bei 37 °C. Anschließend wurden Haftversuche als Abzugsversuche an einer Universalprüfmaschine bei einer Prüfgeschwindigkeit von 1 mm/min in den folgenden vier Gruppen durchgeführt.

a) Die Kronen wurden aus nicht konditionierten Blöcken hergestellt und der Haftversuch wurde direkt nach der Aushärtung durchgeführt.

b) Die Kronen wurden aus nicht konditionierten Blöcken hergestellt und der Haftversuch wurde nach der Aushärtung und einer Wasserlagerung von 8 Wochen bei 37°C durchgeführt.

c) Die Kronen wurden aus konditionierten Blöcken (Wasserlagerung bei 60 °C, bis beim wöchentlichen Test ein Konditionierungsgrad zumindest von 90% erreicht wurde) hergestellt und der Haftversuch wurde nach der Aushärtung direkt durchgeführt.

d) Die Kronen wurden aus konditionierten Blöcken (Wasserlagerung bei 60 °C bis beim wöchentlichen Test ein Konditionierungsgrad von zumindest 90% erreicht wurde) hergestellt und der Haftversuch wurde nach der Aushärtung und einer Wasserlagerung von 8 Wochen bei 37°C durchgeführt.

Partikelgrößenbestimmung:

Die Partikelgrößenbestimmung der Nanopartikel "Nano-SiO2" erfolgte mittels Dynamic light scattering (DLS) mit einem Gerät des Typs Zetasizer Nano ZS (Malvern) bei 0,5 Gew.-% in 2-Butanon (Volumengewichtung).

Die Partikelgrößenbestimmung der Mikropartikel (Dentalglas) erfolgte mittels Laserbeugung unter Verwendung eines Geräts des Typs Beckmann Coulter LS 13320.

Tabelle 1:

| Referenzen | Lava Ultimate (3M Espe) | Cerasmart (GC) | Block HC (Shofu) | Crios (Coltene) |
|---|---|---|---|---|
| Füllstoffgehalt (Herstellerangabe) [%] | 80 | | | 70.7 |
| Glührückstand [%] | 73 | 65 | 62 | 70 |
| Biaxiale Biegefestigkeit [MPa] | 174 | 214 | 147 | 232 |
| 3-Punkt-Biegefestigkeit [MPa] | 163 | 159 | 122 | 198 |
| E-Modul [GPa] | 11.8 | 9.9 | 8.7 | 12.7 |
| $W_{SP}$ [$\mu$g/mm$^3$] | 36 | 29 | 40 | 23 |
| $W_{SP}$ /E [$\mu$g/(GPa x mm$^3$)] | 3.05 | 2.93 | 4.60 | 1.81 |
| LQ (1 Woche) [%] | 0.23% | 0.19% | 0.27% | 0.15% |
| LQ (2 Wochen) [%] | 0.42% | 0.36% | 0.48% | 0.27% |
| LQ (4 Wochen) [%] | 0.51% | 0.42% | 0.60% | 0.34% |
| LQ (8 Wochen) [%] | 0.53% | 0.44% | 0.63% | 0.35% |

Tabelle 2:

| Beispiel | | | 1 | 2 | 3 | 4 |
|---|---|---|---|---|---|---|
| Füllstoff (a) | (a1a) | Dentalglas 1 | 13.00 | 13.00 | 13.00 | 13.00 |
| | (a1b) | Dentalglas 2 | 57.50 | 57.50 | 57.50 | 57.50 |
| | | Dentalglas 3 | | | | |
| | | Dentalglas 4 | | | | |
| | | Dentalglas 5 | | | | |
| | (a2) | NanO-SiO$_2$ (40 nm) | 15.00 | 15.00 | 15.00 | 15.00 |
| | Gesamt-(a) | | 85.50 | 85.50 | 85.50 | 85.50 |
| Monomere (b) | (b1a) | Bis-EMA2,6 | 6.00 | 6.00 | 6.00 | 6.50 |
| | (b1b) | Bis-EMA4 | | | | |
| | | Bis-EMA6 | | | | |
| | | Bis-EMA10 | | | | |
| | (b2) | TCDDMA | 3.75 | 5.00 | 2.50 | 3.50 |
| | | UDMA | 3.75 | 2.50 | 5.00 | 3.50 |
| | | HDDMA | 0.70 | 0.70 | 0.70 | 0.70 |
| | | DODMA | | | | |
| | | TEGDMA | | | | |
| | Gesamt-(b) | | 14.20 | 14.20 | 14.20 | 14.20 |
| Initiatoren (c) | | BPO | 0.30 | 0.30 | 0.30 | 0.30 |
| Gesamt | | | 100.00 | 100.00 | 100.00 | 100.00 |

Tabelle 3:

| Beispiel | 1 | 2 | 3 | 4 |
|---|---|---|---|---|
| (a1a)/(a1b) | 0.23 | 0.23 | 0.23 | 0.23 |
| (a2)/[(a 1 a)+(a 1 b)] | 0.21 | 0.21 | 0.21 | 0.21 |
| (b1a)/(b)x100% | 42.3% | 42.3% | 42.3% | 45.8% |
| Biaxiale Biegefestigkeit [MPa] | 301 | 269 | 292 | 284 |
| 3-Punkt-Biegefestigkeit [MPa] | 274 | 241 | 266 | 259 |
| E-Modul [GPa] | 18.3 | 15.8 | 18.6 | 16.4 |
| $W_{SP}$ [$\mu$g/mm$^3$] | 13 | 11 | 15 | 12 |
| $W_{SP}$/E [$\mu$g/(GPa x mm$^3$)] | 0.71 | 0.70 | 0.81 | 0.73 |
| LQ (1 Woche) [%] | 0.09% | 0.09% | 0.10% | 0.08% |
| LQ (2 Wochen) [%] | 0.15% | 0.14% | 0.16% | 0.13% |
| LQ (4 Wochen) [%] | 0.19% | 0.18% | 0.19% | 0.17% |
| LQ (8 Wochen) [%] | 0.19% | 0.19% | 0.20% | 0.18% |
| Haftung (Gruppe a) [N] | 530 | 526 | 535 | 527 |
| Haftung (Gruppe b) [N] | 411 | 407 | 408 | 407 |
| Haftung (Gruppe c) [N] | 526 | 512 | 518 | 514 |
| Haftung (Gruppe d) [N] | 501 | 493 | 497 | 496 |

Tabelle 4:

| Beispiel | | | 5 | 6 | 7 | 8 |
|---|---|---|---|---|---|---|
| Füllstoff (a) | (a1a) | Dentalglas 1 | 13.00 | 13.00 | 13.00 | 13.00 |
| | (a1b) | Dentalglas 2 | 57.50 | 57.50 | 57.50 | 57.50 |
| | | Dentalglas 3 | | | | |
| | | Dentalglas 4 | | | | |
| | | Dentalglas 5 | | | | |
| | (a2) | NanO-$SiO_2$ (40 nm) | 15.00 | 15.00 | 15.00 | 15.00 |
| | Gesamt-(a) | | 85.50 | 85.50 | 85.50 | 85.50 |
| Monomere (b) | (b1a) | Bis-EMA2,6 | 7.00 | 5.80 | 6.00 | 6.00 |
| | (b1b) | Bis-EMA4 | | | | |
| | | Bis-EMA6 | | | | |
| | | Bis-EMA10 | | | | |
| | (b2) | TCDDMA | 3.25 | 3.85 | 3.75 | 3.75 |
| | | UDMA | 3.25 | 3.85 | 3.75 | 3.75 |
| | | HDDMA | 0.70 | 0.70 | | |
| | | DODMA | | | 0.70 | |
| | | TEGDMA | | | | 0.70 |
| | Gesamt-(b) | | 14.20 | 14.20 | 14.20 | 14.20 |
| Initiatoren (c) | | BPO | 0.30 | 0.30 | 0.30 | 0.30 |
| Gesamt | | | 100.00 | 100.00 | 100.00 | 100.00 |

Tabelle 5:

| Beispiel | 5 | 6 | 7 | 8 |
|---|---|---|---|---|
| (a1a)/(a1b) | 0.23 | 0.23 | 0.23 | 0.23 |
| (a2)/[(a 1 a)+(a 1 b)] | 0.21 | 0.21 | 0.21 | 0.21 |
| (b1 a)/(b)x100% | 49.3% | 40.8% | 42.3% | 42.3% |
| Biaxiale Biegefestigkeit [MPa] | 271 | 295 | 261 | 299 |
| 3-Punkt-Biegefestigkeit [MPa] | 251 | 270 | 237 | 271 |
| E-Modul [GPa] | 15.1 | 18.7 | 15.1 | 19.1 |
| $W_{SP}$ [$\mu$g/mm$^3$] | 13 | 15 | 11 | 14 |
| $W_{SP}$ /E [$\mu$g/(GPa x mm$^3$)] | 0.86 | 0.80 | 0.73 | 0.73 |
| LQ (1 Woche) [%] | 0.09% | 0.09% | 0.05% | 0.07% |
| LQ (2 Wochen) [%] | 0.18% | 0.17% | 0.11% | 0.13% |
| LQ (4 Wochen) [%] | 0.21% | 0.20% | 0.17% | 0.16% |
| LQ (8 Wochen) [%] | 0.22% | 0.21% | 0.19% | 0.17% |
| Haftung (Gruppe a) [N] | 510 | 523 | 504 | 529 |
| Haftung (Gruppe b) [N] | 403 | 410 | 417 | 409 |
| Haftung (Gruppe c) [N] | 501 | 513 | 498 | 510 |

(fortgesetzt)

| Beispiel | 5 | 6 | 7 | 8 |
|---|---|---|---|---|
| Haftung (Gruppe d) [N] | 491 | 502 | 490 | 495 |

Tabelle 6:

| Beispiel | | | 9 | 10 | 11 | 12 |
|---|---|---|---|---|---|---|
| Füllstoff (a) | (a1a) | Dentalglas 1 | | 13.00 | 13.00 | 13.00 |
| | (a1b) | Dentalglas 2 | 70.50 | 57.50 | 57.50 | 57.50 |
| | | Dentalglas 3 | | | | |
| | | Dentalglas 4 | | | | |
| | | Dentalglas 5 | | | | |
| | (a2) | NanO-SiO$_2$ (40 nm) | 15.00 | 15.00 | 15.00 | 15.00 |
| | Gesamt-(a) | | 85.50 | 85.50 | 85.50 | 85.50 |
| Monomere (b) | (b1a) | Bis-EMA2,6 | 6.00 | | | |
| | (b1b) | Bis-EMA4 | | 6.00 | | |
| | | Bis-EMA6 | | | 6.00 | |
| | | Bis-EMA10 | | | | 6.00 |
| | (b2) | TCDDMA | 3.75 | 3.75 | 3.75 | 3.75 |
| | | UDMA | 3.75 | 3.75 | 3.75 | 3.75 |
| | | HDDMA | 0.70 | 0.70 | 0.70 | 0.70 |
| | | DODMA | | | | |
| | | TEGDMA | | | | |
| | Gesamt-(b) | | 14.20 | 14.20 | 14.20 | 14.20 |
| Initiatoren (c) | | BPO | 0.30 | 0.30 | 0.30 | 0.30 |
| Gesamt | | | 100.00 | 100.00 | 100.00 | 100.00 |

Tabelle 7:

| Beispiel | 9 | 10 | 11 | 12 |
|---|---|---|---|---|
| (a1a)/(a1b) | 0.00 | 0.23 | 0.23 | 0.23 |
| (a2)/[(a 1 a)+(a 1 b)] | 0.21 | 0.21 | 0.21 | 0.21 |
| (b1 a)/(b)x1 00% | 42.3% | 42.3% | 0.0% | 0.0% |
| Biaxiale Biegefestigkeit [MPa] | 256 | 289 | 262 | 219 |
| 3-Punkt-Biegefestigkeit [MPa] | 221 | 262 | 239 | 198 |
| E-Modul [GPa] | 13.4 | 17.7 | 13.7 | 12.6 |
| W$_{SP}$ [$\mu$g/mm$^3$] | 18 | 15 | 19 | 26 |
| W$_{SP}$ /E [$\mu$g/(GPa x mm$^3$)] | 1.34 | 0.85 | 1.39 | 2.06 |
| LQ (1 Woche) [%] | 0.13% | 0.10% | 0.15% | 0.17% |
| LQ (2 Wochen) [%] | 0.22% | 0.16% | 0.27% | 0.29% |
| LQ (4 Wochen) [%] | 0.28% | 0.21% | 0.31% | 0.36% |

(fortgesetzt)

| Beispiel | 9 | 10 | 11 | 12 |
|---|---|---|---|---|
| LQ (8 Wochen) [%] | 0.28% | 0.22% | 0.32% | 0.38% |
| Haftung (Gruppe a) [N] | 511 | 513 | 509 | 508 |
| Haftung (Gruppe b) [N] | 377 | 398 | 299 | 87 |
| Haftung (Gruppe c) [N] | 497 | 502 | 493 | 478 |
| Haftung (Gruppe d) [N] | 472 | 488 | 451 | 412 |

Tabelle 8:

| Beispiel | | | 13 | 14 | 15 | 16 |
|---|---|---|---|---|---|---|
| Füllstoff (a) | (a1a) | Dentalglas 1 | 12.70 | 12.40 | 13.00 | 12.40 |
| | (a1b) | Dentalglas 2 | 56.10 | 54.70 | 57.50 | 54.70 |
| | | Dentalglas 3 | | | | |
| | | Dentalglas 4 | | | | |
| | | Dentalglas 5 | | | | |
| | (a2) | NanO-SiO$_2$ (40 nm) | 14.60 | 14.20 | 15.00 | 14.20 |
| | Gesamt-(a) | | 83.40 | 81.30 | 85.50 | 81.30 |
| Monomere (b) | (b1a) | Bis-EMA2,6 | 6.90 | 7.80 | 5.00 | 6.40 |
| | | Bis-EMA4 | | | | |
| | (b1b) | Bis-EMA6 | | | | |
| | | Bis-EMA10 | | | | |
| | (b2) | TCDDMA | 4.30 | 4.85 | 3.75 | 6.00 |
| | | UDMA | 4.30 | 4.85 | 3.75 | 6.00 |
| | | HDDMA | 0.80 | 0.90 | 1.70 | |
| | | DODMA | | | | |
| | | TEGDMA | | | | |
| | Gesamt-(b) | | 16.30 | 18.40 | 14.20 | 18.40 |
| Initiatoren (c) | | BPO | 0.30 | 0.30 | 0.30 | 0.30 |
| Gesamt | | | 100.00 | 100.00 | 100.00 | 100.00 |

Tabelle 9:

| Beispiel | 13 | 14 | 15 | 16 |
|---|---|---|---|---|
| (a1a)/(a1b) | 0.23 | 0.23 | 0.23 | 0.23 |
| (a2)/[(a1a)+(a1b)] | 0.21 | 0.21 | 0.21 | 0.21 |
| (b1a)/(b)×100% | 42.3% | 42.4% | 35.2% | 34.8% |
| Biaxiale Biegefestigkeit [MPa] | 266 | 201 | 189 | 203 |
| 3-Punkt-Biegefestigkeit [MPa] | 240 | 175 | 169 | 177 |
| E-Modul [GPa] | 16.7 | 13.7 | 11.8 | 12.1 |
| $W_{SP}$ [$\mu$g/mm$^3$] | 15 | 19 | 16 | 17 |

(fortgesetzt)

| Beispiel | 13 | 14 | 15 | 16 |
|---|---|---|---|---|
| $W_{SP}$ /E [µg/(GPa x mm$^3$)] | 0.90 | 1.39 | 1.36 | 1.40 |
| LQ (1 Woche) [%] | 0.10% | 0.16% | 0.14% | 0.14% |
| LQ (2 Wochen) [%] | 0.17% | 0.26% | 0.24% | 0.26% |
| LQ (4 Wochen) [%] | 0.23% | 0.31% | 0.32% | 0.31% |
| LQ (8 Wochen) [%] | 0.23% | 0.31% | 0.32% | 0.32% |
| Haftung (Gruppe a) [N] | 514 | 507 | 500 | 502 |
| Haftung (Gruppe b) [N] | 379 | 274 | 281 | 277 |
| Haftung (Gruppe c) [N] | 497 | 483 | 485 | 488 |
| Haftung (Gruppe d) [N] | 485 | 443 | 456 | 440 |

Tabelle 10:

| Beispiel | | | 17 | 18 | 19 | 20 |
|---|---|---|---|---|---|---|
| Füllstoff (a) | (a1a) | Dentalglas 1 | 13.00 | 12.40 | 12.70 | 12.70 |
| | (a1b) | Dentalglas 2 | 57.50 | | | |
| | | Dentalglas 3 | | 54.70 | | |
| | | Dentalglas 4 | | | 56.10 | |
| | | Dentalglas 5 | | | | 56.10 |
| | (a2) | NanO-SiO$_2$ (40 nm) | 15.00 | 14.20 | 14.60 | 14.60 |
| | Gesamt-(a) | | 85.50 | 81.30 | 83.40 | 83.40 |
| Monomere (b) | (b1a) | Bis-EMA2,6 | 7.50 | 7.80 | 6.90 | 6.90 |
| | (b1b) | Bis-EMA4 | | | | |
| | | Bis-EMA6 | | | | |
| | | Bis-EMA10 | | | | |
| | (b2) | TCDDMA | 3.00 | 4.85 | 4.30 | 4.30 |
| | | UDMA | 3.00 | 4.85 | 4.30 | 4.30 |
| | | HDDMA | 0.70 | 0.90 | 0.80 | 0.80 |
| | | DODMA | | | | |
| | | TEGDMA | | | | |
| | Gesamt-(b) | | 14.20 | 18.40 | 16.30 | 16.30 |
| Initiatoren (c) | | BPO | 0.30 | 0.30 | 0.30 | 0.30 |
| Gesamt | | | 100.00 | 100.00 | 100.00 | 100.00 |

Tabelle 11:

| Beispiel | 17 | 18 | 19 | 20 |
|---|---|---|---|---|
| (a1a)/(a1b) | 0.23 | 0.23 | 0.23 | 0.23 |
| (a2)/[(a1a)+(a1b)] | 0.21 | 0.21 | 0.21 | 0.21 |
| (b1a)/(b)x100% | 52.8% | 42.4% | 42.3% | 42.3% |

(fortgesetzt)

| Beispiel | 17 | 18 | 19 | 20 |
|---|---|---|---|---|
| Biaxiale Biegefestigkeit [MPa] | 198 | 213 | 265 | 231 |
| 3-Punkt-Biegefestigkeit [MPa] | 177 | 194 | 242 | 211 |
| E-Modul [GPa] | 11.0 | 14.4 | 14.7 | 12.5 |
| $W_{SP}$ [$\mu$g/mm$^3$] | 15 | 20 | 16 | 17 |
| $W_{SP}$ /E [$\mu$g/(GPa x mm$^3$)] | 1.36 | 1.39 | 1.09 | 1.36 |
| LQ (1 Woche) [%] | 0.16% | 0.18% | 0.12% | 0.14% |
| LQ (2 Wochen) [%] | 0.25% | 0.27% | 0.19% | 0.23% |
| LQ (4 Wochen) [%] | 0.30% | 0.31% | 0.25% | 0.30% |
| LQ (8 Wochen) [%] | 0.31% | 0.31% | 0.26% | 0.31% |
| Haftung (Gruppe a) [N] | 503 | 502 | 506 | 500 |
| Haftung (Gruppe b) [N] | 283 | 271 | 337 | 274 |
| Haftung (Gruppe c) [N] | 493 | 498 | 495 | 493 |
| Haftung (Gruppe d) [N] | 447 | 423 | 472 | 439 |

Tabelle 12:

| Beispiel | | | 21 | 22 | 23 | 24 |
|---|---|---|---|---|---|---|
| Füllstoff (a) | (a1a) | Dentalglas 1 | 35.25 | 7.00 | 10.00 | 13.92 |
| | (a1b) | Dentalglas 2 | 35.25 | 60.10 | 44.20 | 61.58 |
| | | Dentalglas 3 | | | | |
| | | Dentalglas 4 | | | | |
| | | Dentalglas 5 | | | | |
| | (a2) | NanO-SiO$_2$ (40 nm) | 15.00 | 14.20 | 25.00 | 10.00 |
| | Gesamt-(a) | | 85.50 | 81.30 | 79.20 | 85.50 |
| Monomere (b) | (b1a) | Bis-EMA2,6 | 6.00 | 7.80 | 8.70 | 6.00 |
| | | Bis-EMA4 | | | | |
| | (b1b) | Bis-EMA6 | | | | |
| | | Bis-EMA10 | | | | |
| | (b2) | TCDDMA | 3.75 | 4.85 | 5.40 | 3.75 |
| | | UDMA | 3.75 | 4.85 | 5.40 | 3.75 |
| | | HDDMA | 0.70 | 0.90 | 1.00 | 0.70 |
| | | DODMA | | | | |
| | | TEGDMA | | | | |
| | Gesamt-(b) | | 14.20 | 18.40 | 20.50 | 14.20 |
| Initiatoren (c) | | BPO | 0.30 | 0.30 | 0.30 | 0.30 |
| Gesamt | | | 100.00 | 100.00 | 100.00 | 100.00 |

Tabelle 13:

| Beispiel | 21 | 22 | 23 | 24 |
|---|---|---|---|---|
| (a1a)/(a1b) | 1.00 | 0.12 | 0.23 | 0.23 |
| (a2)/[(a1a)+(a1b)] | 0.21 | 0.21 | 0.46 | 0.13 |
| (b1a)/(b)x100% | 42.3% | 42.4% | 42.4% | 42.3% |
| Biaxiale Biegefestigkeit [MPa] | 204 | 197 | 156 | 188 |
| 3-Punkt-Biegefestigkeit [MPa] | 181 | 174 | 128 | 145 |
| E-Modul [GPa] | 11.7 | 14.3 | 11.7 | 12.4 |
| $W_{SP}$ [$\mu$g/mm$^3$] | 19 | 21 | 31 | 21 |
| $W_{SP}$/E [$\mu$g/(GPa x mm$^3$)] | 1.62 | 1.47 | 2.65 | 1.69 |
| LQ (1 Woche) [%] | 0.19% | 0.20% | 0.23% | 0.21% |
| LQ (2 Wochen) [%] | 0.27% | 0.26% | 0.37% | 0.29% |
| LQ (4 Wochen) [%] | 0.32% | 0.31% | 0.43% | 0.34% |
| LQ (8 Wochen) [%] | 0.33% | 0.32% | 0.45% | 0.35% |
| Haftung (Gruppe a) [N] | 504 | 513 | 503 | 505 |
| Haftung (Gruppe b) [N] | 188 | 204 | 53 | 127 |
| Haftung (Gruppe c) [N] | 481 | 488 | 473 | 484 |
| Haftung (Gruppe d) [N] | 421 | 438 | 382 | 417 |

Tabelle 14:

| Beispiel | | | 25 | 26 | | |
|---|---|---|---|---|---|---|
| Füllstoff (a) | (a1a) | Dentalglas 1 | 15.00 | 13.50 | | |
| | (a1b) | Dentalglas 2 | 39.50 | 35.50 | | |
| | | Dentalglas 3 | | | | |
| | | Dentalglas 4 | | | | |
| | | Dentalglas 5 | | | | |
| | (a2) | Nano-SiO$_2$ (40 nm) | 17.00 | 15.35 | | |
| | Gesamt-(a) | | 71.50 | 64.35 | | |
| Monomere (b) | (b1a) | Bis-EMA2,6 | 12.20 | 15.25 | | |
| | | Bis-EMA4 | | | | |
| | (b1b) | Bis-EMA6 | | | | |
| | | Bis-EMA10 | | | | |
| | (b2) | TCDDMA | 8.00 | 10.00 | | |
| | | UDMA | 8.00 | 10.00 | | |
| | | HDDMA | | | | |
| | | DODMA | | | | |
| | | TEGDMA | | | | |
| | Gesamt-(b) | | 28.20 | 35.25 | | |
| Initiatoren (c) | | BPO | 0.30 | 0.30 | | |
| Gesamt | | | 100.00 | 100.00 | | |

Tabelle 15:

| Beispiel | 25 | 26 | Keramik (Vergleich) | |
|---|---|---|---|---|
| (a1a)/(a1b) | 0.38 | 0.38 | n.a. | |
| (a2)/[(a 1 a)+(a 1 b)] | 0.31 | 0.31 | n.a. | |
| (b1a)/(b)×100% | 43.3% | 43.3% | n.a. | |
| Biaxiale Biegefestigkeit [MPa] | 220 | 150 | 360 | |
| 3-Punkt-Biegefestigkeit [MPa] | 201 | 129 | | |
| E-Modul [GPa] | 11.1 | 8.7 | 95 | |
| $W_{SP}$ [$\mu$g/mm$^3$] | 20 | 41 | | |
| $W_{SP}$ /E [$\mu$g/(GPa x mm$^3$)] | 1.80 | 4.71 | | |
| LQ (1 Woche) [%] | 0.13% | 0.26% | | |
| LQ (2 Wochen) [%] | 0.25% | 0.53% | | |
| LQ (4 Wochen) [%] | 0.33% | 0.62% | | |
| LQ (8 Wochen) [%] | 0.34% | 0.65% | | |
| Haftung (Gruppe a) [N] | 516 | 501 | 547 | |
| Haftung (Gruppe b) [N] | 98 | 0* | 539 | |
| Haftung (Gruppe c) [N] | 491 | 456 | 544 | |
| Haftung (Gruppe d) [N] | 371 | 321 | 540 | |
| * Debonding bei allen Kronen während der Wasserlagerung Im Folgenden wurde der Konditionierungsgrad für Kompositblöcke gemäß Beispiel 1 wie oben beschrieben zeitabhängig bei 37 °C und 60 °C bestimmt. | | | | |

Tabelle 16 (zeitabhängiger Konditionierungsgrad von Beispiel 1):

| | 37 °C | 60 °C |
|---|---|---|
| Konditionierungsgrad (1 Woche) [%] | 11% | 44% |
| Konditionierungsgrad (2 Wochen) [%] | 16% | 65% |
| Konditionierungsgrad (3 Wochen) [%] | 23% | 76% |
| Konditionierungsgrad (4 Wochen) [%] | 25% | 82% |
| Konditionierungsgrad (5 Wochen) [%] | 28% | 85% |
| Konditionierungsgrad (6 Wochen) [%] | 31% | 87% |
| Konditionierungsgrad (7 Wochen) [%] | 34% | 89% |
| Konditionierungsgrad (8 Wochen) [%] | 36% | 91%* |
| Konditionierungsgrad (12 Wochen) [%] | 44% | 93% |
| Konditionierungsgrad (16 Wochen) [%] | 52% | 94% |
| Konditionierungsgrad (20 Wochen) [%] | 59% | 95% |
| * Messungen zur Haftung (Gruppen c und d) gemäß Tabelle 3 bei diesem Konditionierungsgrad durchgeführt | | |

Tabelle 17 (zeitabhängiger Konditionierungsgrad von Beispiel 25):

| | 37 °C | 60 °C |
|---|---|---|
| Konditionierungsgrad (1 Woche) [%] | 12% | 46% |

(fortgesetzt)

|  | 37 °C | 60 °C |
|---|---|---|
| Konditionierungsgrad (2 Wochen) [%] | 18% | 66% |
| Konditionierungsgrad (3 Wochen) [%] | 25% | 77% |
| Konditionierungsgrad (4 Wochen) [%] | 27% | 83% |
| Konditionierungsgrad (5 Wochen) [%] | 30% | 85% |
| Konditionierungsgrad (6 Wochen) [%] | 32% | 87% |
| Konditionierungsgrad (7 Wochen) [%] | 35% | 89% |
| Konditionierungsgrad (8 Wochen) [%] | 38% | 91%* |
| Konditionierungsgrad (12 Wochen) [%] | 43% | 92% |
| Konditionierungsgrad (16 Wochen) [%] | 51% | 93% |
| Konditionierungsgrad (20 Wochen) [%] | 57% | 94% |
| * Messungen zur Haftung (Gruppen c und d) gemäß Tabelle 15 bei diesem Konditionierungsgrad durchgeführt | | |

Tabelle 18 (zeitabhängiger Konditionierungsgrad von Beispiel 26):

|  | 37 °C | 60 °C |
|---|---|---|
| Konditionierungsgrad (1 Woche) [%] | 15% | 50% |
| Konditionierungsgrad (2 Wochen) [%] | 20% | 70% |
| Konditionierungsgrad (3 Wochen) [%] | 27% | 79% |
| Konditionierungsgrad (4 Wochen) [%] | 29% | 84% |
| Konditionierungsgrad (5 Wochen) [%] | 32% | 86% |
| Konditionierungsgrad (6 Wochen) [%] | 34% | 87% |
| Konditionierungsgrad (7 Wochen) [%] | 35% | 88% |
| Konditionierungsgrad (8 Wochen) [%] | 38% | 90%* |
| Konditionierungsgrad (12 Wochen) [%] | 42% | 91% |
| Konditionierungsgrad (16 Wochen) [%] | 49% | 92% |
| Konditionierungsgrad (20 Wochen) [%] | 55% | 93% |
| * Messungen zur Haftung (Gruppen c und d) gemäß Tabelle 15 bei diesem Konditionierungsgrad durchgeführt | | |

Ergänzende Untersuchungen:

Ausgehend von den Untersuchungen gemäß den Beispielen 1, 25 und 26 wurden ergänzende Untersuchungen durchgeführt, um die Abhängigkeit bestimmter Messergebnisse für ausgewählte Parameter vom Konditionierungsgrad zu ermitteln. Ergebnisse der weiteren Untersuchungen sind in den nachfolgenden Tabellen 19, 20 und 21 angegeben. Die Tabellen enthalten in der jeweiligen Spalte mit der Überschrift "unkonditioniert" sowie in der jeweils ganz rechts angeordneten Spalte mit der Überschrift "91%" bzw. "90%" Werte, die sich bereits in den Tabellen 3 (für Beispiel 1) bzw. 15 (für die Beispiele 25 bzw. 26) finden. Für die Spalte "unkonditioniert" gibt es eine entsprechende Übereinstimmung für die Zeilen von "3-Punkt-Biegefestigkeit" bis "Haftung (Gruppe b)". Für die Spalte "91%" bzw. "90%" gibt es eine Übereinstimmung hinsichtlich der Zeilen "Haftung (Gruppe c)" sowie "Haftung (Gruppe d)".

Sämtliche weiteren Einträge in den Tabellen 19 bis 21 geben Ergebnisse der ergänzenden Untersuchungen wieder.

Für die ergänzenden Untersuchungen wurden Kompositblöcke bei 60 °C in Wasser gelagert, bis bei den wöchentlichen Tests (siehe oben die Ausführungen zur Wasserbehandlung/-konditionierung der Kompositblöcke und Bestimmung des Konditionierungsgrades) ein jeweiliger Konditionierungsgrad von (zumindest) 25%, 50%, 75% bzw.

**EP 3 685 798 B1**

90% erreicht wurde. Die entsprechenden Kompositblöcke mit den eingestellten Konditionierungsgraden wurden dann den jeweiligen Messungen unterzogen.

Die Tabellen 19 bis 21 zeigen, dass die 3-Punkt-Biegefestigkeit und der E-Modul jeweils keine signifikante Abhängigkeit vom Konditionierungsgrad besitzen.

Die Wasseraufnahme ("$W_{SP}$") (bestimmt analog EN ISO 4049:2009 (D) (7.12)) ist eine vom tatsächlichen Wassergehalt bzw. Konditionierungsgrad eines Kompositblocks unabhängige Eigenschaft, denn gemäß Messmethode wird ein jeder Probekörper zunächst bis zur Massenkonstanz getrocknet und die Wasseraufnahme dann auf diesen getrockneten Referenzzustand bezogen. Daher ist in den Tabellen 19 bis 21 der jeweilige Wert "$W_{SP}$" immer gleich (variiert aber natürlich zwischen den Beispielen 1, 25 und 26).

Die Tabellen 19 bis 21 enthalten jeweils auch Messergebnisse zum Parameter "LQ (8 Wochen)". Soweit hier Untersuchungen mit vorkonditionierten Kompositblöcken durchgeführt wurden (25%, 65%, 76% bzw. 91%), wurde in Abweichung von der weiter oben angegebenen allgemeinen Bestimmungsmethode der jeweilige (vorkonditionierte) Probekörper nicht im Exsikkator bis zur Massenkonstanz getrocknet, sondern direkt der Wasserlagerung zugeführt.

Die Resultate gemäß den Tabellen 19 bis 21 bestätigen, dass vorkonditionierte Kompositblöcke nicht mehr so stark linear quellen wie ein unkonditionierter Kompositblock. Das Quellverhalten ist somit nicht unabhängig vom Grad der Vorkonditionierung, sondern es nimmt mit zunehmender Vorkonditionierung ab.

Hinsichtlich der Untersuchungen zur Haftung ist Folgendes festzustellen:

Ein Vergleich zwischen Gruppe a) und Gruppe b) zeigt jeweils, dass unkonditionierte Blöcke bei Wasserlagerung an Haftqualität verlieren (die Zusammensetzung gemäß Beispiel 1 ist dabei weniger anfällig als die Zusammensetzungen gemäß den Beispielen 25 und 26). Durch Vorkonditionierung (auf Konditionierungsgrade von 25%, 65%, 76% bzw. 91%), das heißt beim Übergang von Gruppe a) zu Gruppe c), werden die erreichten Haftungswerte nicht signifikant beeinträchtigt, das heißt vorkonditionierte Kompositblöcke haben eine ähnlich gute Direkthaftung wie nicht konditionierte Kompositblöcke.

Setzt man gemäß Gruppen b) und d) unkonditionierte bzw. vorkonditionierte Blöcke ein und lagert die daraus hergestellten idealisierten Kronen nach Befestigung am Abutment für 8 Wochen bei 37 °C in Wasser, so ergibt sich im Vergleich mit den Gruppen a) bzw. c), dass der Haftungswert für die nicht konditionierten Blöcke durch Wasserlagerung stark abfällt (zum Beispiel gemäß Tabelle 19 für Beispiel 1 von 530 N (Gruppe a, ohne Wasserlagerung) auf 411 N (Gruppe b, mit Wasserlagerung)), während eine solche Wasserlagerung bei Einsatz von vorkonditionierten Blöcken deutlich weniger nachteilig ist. Jeweils am besten behalten die Kompositblöcke die Hafteigenschaften bei, welche auf einen Konditionierungsgrad von 90 bzw. 91% vorkonditioniert wurden, vgl. Tabelle 19 für Beispiel 1: ein Haftwert von 526 N (ohne Wasserlagerung nach Befestigung der Krone) steht einem Wert von immer noch hervorragenden 501 N entgegen (bestimmt nach Wasserlagerung von 8 Wochen nach Befestigung der Krone). Die Tabellen zeigen somit eindrucksvoll, dass eine Vorkonditionierung dazu führt, dass eine entsprechende Krone nach ihrer Befestigung und nach einer Wasserlagerung von 8 Wochen deutlich besser am entsprechenden Abutment anhaftet als eine Krone aus einem nicht vorkonditionierten Kompositblock.

Tabelle 19 (Beispiel 1 bei unterschiedlichem Konditionierungsgrad):

| Konditionierungsgrad [%] | unkonditioniert | 25% | 65% | 76% | 91% |
|---|---|---|---|---|---|
| 3-Punkt-Biegefestigkeit [MPa] | 274 | 276 | 271 | 269 | 270 |
| E-Modul [GPa] | 18.3 | 18.3 | 18.2 | 18.1 | 17.9 |
| $W_{SP}$ [$\mu$g/mm$^3$] | 13 | 13 | 13 | 13 | 13 |
| LQ (8 Wochen) [%] | 0.19% | 0.15% | 0.07% | 0.05% | 0.02% |
| Haftung (Gruppe a) [N] | 530 | n.a. | n.a. | n.a. | n.a. |
| Haftung (Gruppe b) [N] | 411 | n.a. | n.a. | n.a. | n.a. |
| Haftung (Gruppe c) [N] | n.a. | 525 | 527 | 522 | 526 |
| Haftung (Gruppe d) [N] | n.a. | 439 | 468 | 489 | 501 |

Tabelle 20 (Beispiel 25 bei unterschiedlichem Konditionierungsgrad):

| Konditionierungsgrad [%] | unkonditioniert | 25% | 66% | 77% | 91% |
|---|---|---|---|---|---|
| 3-Punkt-Biegefestigkeit [MPa] | 201 | 195 | 202 | 193 | 198 |
| E-Modul [GPa] | 11.1 | 11.1 | 11.0 | 10.8 | 10.5 |

30

(fortgesetzt)

| Konditionierungsgrad [%] | unkonditioniert | 25% | 66% | 77% | 91% |
|---|---|---|---|---|---|
| $W_{SP}$ [$\mu$g/mm$^3$] | 20 | 20 | 20 | 20 | 20 |
| LQ (8 Wochen) [%] | 0.34% | 0.27% | 0.13% | 0.09% | 0.04% |
| Haftung (Gruppe a) [N] | 516 | n.a. | n.a. | n.a. | n.a. |
| Haftung (Gruppe b) [N] | 98 | n.a. | n.a. | n.a. | n.a. |
| Haftung (Gruppe c) [N] | n.a. | 510 | 501 | 498 | 491 |
| Haftung (Gruppe d) [N] | n.a. | 128 | 278 | 339 | 371 |

Tabelle 21 (Beispiel 26 bei unterschiedlichem Konditionierungsgrad):

| Konditionierungsgrad [%] | unkonditioniert | 27% | 50% | 79% | 90% |
|---|---|---|---|---|---|
| 3-Punkt-Biegefestigkeit [MPa] | 129 | 127 | 123 | 118 | 109 |
| E-Modul [GPa] | 8.7 | 8.6 | 8.4 | 8.1 | 7.5 |
| $W_{SP}$ [$\mu$g/mm$^3$] | 41 | 41 | 41 | 41 | 41 |
| LQ (8 Wochen) [%] | 0.65% | 0.50% | 0.34% | 0.15% | 0.08% |
| Haftung (Gruppe a) [N] | 501 | n.a. | n.a. | n.a. | n.a. |
| Haftung (Gruppe b) [N] | 0 | n.a. | n.a. | n.a. | n.a. |
| Haftung (Gruppe c) [N] | n.a. | 496 | 482 | 473 | 456 |
| Haftung (Gruppe d) [N] | n.a. | 39 | 117 | 222 | 321 |

**Patentansprüche**

1. Fräsrohling zur Herstellung einer indirekten dentalen Restauration,

   aus Kunststoff oder einem kunststoffbasierten Komposit,
   umfassend Wasser in einer Menge von wenigstens 25 % der Wasseraufnahme $W_{sp}$, wobei die Wasseraufnahme ($W_{SP}$) analog EN ISO 4049:2009 [D] [7.12] bestimmt wird;
   nachfolgend werden die Bestimmungsmethode zusammengefasst und Abweichungen von der Vorgehensweise gemäß EN ISO 4049:2009 [D] angegeben: Aus hergestellten Komposit-Blöcken werden Probekörper mit einer Länge von 14,7 mm, einer Breite von 14,7 mm und einer Dicke von 0,5 mm mit einer Hochgeschwindigkeitssäge hergestellt, entgratet, geschliffen und poliert; die Probekörper werden [analog 7.12.3.1] im Exsikkator bei 37 °C bis zur Massenkonstanz getrocknet und die Masse ($m_1$) auf 0,1 mg, sowie [analog 7.12.3.2] die Länge, die Breite und die Dicke auf 0,01 mm genau bestimmt; hieraus wird das Volumen V in Kubikmillimeter berechnet; anschließend werden die Probekörper [analog 7.12.3.3] für 7 Tage bei 37 °C in Wasser gelagert; nach 7 Tagen werden die Probekörper herausgenommen, mit Wasser abgespült, abgetupft, für 15 Sekunden in der Luft hin- und hergeschwenkt und 1 Minute nach dem Herausnehmen aus dem Wasser auf 0,1 mg genau gewogen ($m_2$); nach dieser Wägung werden [analog 7.12.3.4] die Probekörper im Exsikkator bei 37 °C bis zur Massenkonstanz getrocknet und die Masse ($m_3$) auf 0,1 mg bestimmt; die Wasseraufnahme wird entsprechend der unter 7.12.4.1 angegebenen Gleichung (2) berechnet.

2. Fräsrohling nach Anspruch 1,

   umfassend Wasser in einer Menge von wenigstens 50 %, bevorzugt wenigstens 75 %, besonders bevorzugt wenigstens 90 % der Wasseraufnahme $W_{sp}$
   und/oder
   mit einem E-Modul, bestimmt nach der ADA Specification No. 27 - 1993, von wenigstens 10 GPa, vorzugsweise von wenigstens 13 GPa und besonders bevorzugt von wenigstens 15 GPa
   und/oder mit einer Wasseraufnahme $W_{sp}$ von höchstens 40 µg/mm$^3$, vorzugsweise von höchstens 30 µg/mm$^3$ und besonders bevorzugt von höchstens 20 µg/mm$^3$.

3. Fräsrohling nach einem der vorangehenden Ansprüche, wobei der Quotient aus der Wasseraufnahme $W_{sp}$ und dem nach der ADA Specification No. 27 - 1993 bestimmten E-Modul kleiner ist als 1,35 µg/(GPa x mm$^3$), vorzugsweise kleiner ist als 1,00 µg/(GPa × mm$^3$).

4. Fräsrohling nach einem der vorangehenden Ansprüche, aus kunststoffbasiertem Komposit, umfassend

   a) einen anorganischen Füllstoff in einer Menge von wenigstens 70 Gew.-%, vorzugsweise wenigstens 80 Gew.-%, bezogen auf die Gesamtmasse des Fräsrohlings
   und
   b) eine Harzmatrix.

5. Fräsrohling nach Anspruch 4, wobei der anorganische Füllstoff a) umfasst:

   a1) eine Glaszusammensetzung und
   a2) nicht aggregierte und nicht agglomerierte Kieselsäure mit einer mittleren Teilchengröße von nicht mehr als 80 nm.

6. Fräsrohling nach Anspruch 5, wobei die Glaszusammensetzung a1)

   eine erste Glaszusammensetzung a1a) mit einem D50-Wert im Bereich von 0,4 bis 1,0 µm, vorzugsweise im Bereich von 0,5 bis 0,9 µm,
   und
   eine zweite Glaszusammensetzung a1b) mit einem D50-Wert im Bereich von 1,2 bis 5,0 µm, vorzugsweise im Bereich von 1,5 bis 4,0 µm,
   umfasst,
   wobei das Massenverhältnis von a1a) zu a1b) zwischen 1 : 1,5 und 1 : 8, vorzugsweise zwischen 1 : 2 und 1 : 5 liegt,
   wobei das Massenverhältnis von a2) zur Summe von a1a) und a1b) zwischen 1 : 3 und 1 : 6 liegt,
   wobei das Verhältnis des D50-Werts der ersten Glaszusammensetzung a1a) zum D50-Wert der zweiten Glaszusammensetzung a1b) im Bereich von 1 : 1,5 bis 1 : 10, vorzugsweise 1 : 2 bis 1 : 5, liegt,
   und wobei der D75-Wert der ersten Glaszusammensetzung a1a) kleiner ist als der D25-Wert der zweiten Glaszusammensetzung a1b).

7. Fräsrohling nach einem der Ansprüche 4 bis 6, wobei die Harzmatrix b) ein Polymerisat von Monomeren ist, die difunktionelle (Meth)acrylate enthalten, wobei der Gewichtsanteil von ethoxyliertem Bisphenol-A-Dimethacrylat mit einem durchschnittlichen Ethoxylierungsgrad von 2 bis 4 Ethoxygruppen pro Molekül größer ist als 40 Gew.-% und kleiner ist als 50 Gew.-%, bezogen auf die Gesamtmasse der Monomere.

8. Fräsrohling nach einem der vorangehenden Ansprüche, wobei die Differenz zwischen der Wasseraufnahme $W_{sp}$ und dem Wassergehalt des Fräsrohlings kleiner ist als 10 µg/mm$^3$, vorzugsweise kleiner ist als 8 µg/mm$^3$, bevorzugt kleiner ist als 4 µg/mm$^3$.

9. Fräsrohling nach einem der vorangehenden Ansprüche, dessen Abmessungen so gewählt sind, dass aus ihm

   - ein Würfel mit einer Kantenlänge von 10 mm, vorzugsweise 14 mm,
   und/oder
   - ein Quader mit einer quadratischen Grundfläche mit einer Kantenlänge von 10mm, vorzugsweise 14 mm, und einer Höhe von 20 mm
   fräsbar ist.

10. Fräsrohling nach einem der vorangehenden Ansprüche, wobei der Fräsrohling wasserdicht versiegelt oder umschlossen ist, vorzugsweise umschlossen ist von einem wasserdichten Behältnis, vorzugsweise einem wasserdichten Blister, und/oder einem wasserdichten Lack und/oder einer wasserdichten Hülle aus Wachs.

11. Verwendung eines Fräsrohlings nach einem der vorangehenden Ansprüche zur Herstellung eines Formkörpers zur Verwendung als indirekte dentale Restauration, vorzugsweise eines Formkörpers ausgewählt aus der Gruppe bestehend aus Inlay, Onlay, Teilkrone, Krone, Veneer und Brücke.

**12.** Verfahren zur Herstellung eines Fräsrohlings nach einem der Ansprüche 1 bis 10 oder eines daraus gefertigten Formkörpers zur Verwendung als indirekte dentale Restauration, mit folgenden Schritten:

(i) Herstellen oder Bereitstellen eines Fräsrohlings umfassend Wasser in einer Menge von weniger als 25 % der Wasseraufnahme $W_{sp}$, bevorzugt von weniger als 15 %, besonders bevorzugt von weniger als 10 %,

(ii) Einstellen von Bedingungen, bei denen der in Schritt (i) bereitgestellte oder hergestellte Fräsrohling Wasser aufnimmt und Beibehalten dieser Bedingungen, bis der Fräsrohling Wasser in einer Menge von wenigstens 25 % der Wasseraufnahme $W_{sp}$ umfasst, sowie vorzugsweise wasserdichtes Versiegeln oder Umschließen des erzeugten Fräsrohlings.

**13.** Verfahren nach Anspruch 12 zur Herstellung eines Formkörpers zur Verwendung als indirekte dentale Restauration, umfassend den zusätzlichen Schritt:

(iii) Fräsen des Formkörpers aus dem in Schritt (ii) erzeugten Fräsrohling, vorzugsweise mittels einer CAD/CAM-Fräseinrichtung, sowie vorzugsweise Polieren des Formkörpers.

**14.** Kit zur Herstellung von indirekten dentalen Restaurationen, umfassend

- zwei oder mehr Fräsrohlinge nach einem der Ansprüche 1 bis 10, in unterschiedlichen Farben
sowie
- ein oder mehrere dentale Zusammensetzungen zur Befestigung eines aus einem der Fräsrohlinge gefrästen Formkörpers an einem dentalen Objekt in der Mundhöhle, vorzugsweise ausgewählt aus der Gruppe bestehend aus Primern, Adhäsiven und Befestigungszementen,
sowie vorzugsweise
- weiteres Zubehör wie Pinsel, Poliermittel und Mischkanülen.

## Claims

**1.** Milling blank for producing an indirect dental restoration,
composed of resin or a resin-based composite, comprising water in an amount of at least 25% of the water sorption $W_{sp}$, wherein water sorption ($W_{sp}$) is determined analogously to EN ISO 4049:2009 [D] [7.12]; there follows a summary of the determination method and specification of the departures from the procedure according to EN ISO 4049:2009 [D]:
A high-speed saw is used to produce specimens with a length of 14.7 mm, a width of 14.7 mm and a thickness of 0.5 mm from composite blocks produced, and these test specimens are deburred, ground, and polished; the specimens [analogously to 7.12.3.1] are dried in a desiccator at 37°C to constant mass and the mass ($m_1$) is determined accurately to 0.1 mg, and [analogously to 7.12.3.2] the length, the width and the thickness are determined accurately to 0.01 mm; the volume V in cubic millimetres is determined from this; subsequently, the test specimens [analogously to 7.12.3.3] are stored for 7 days at 37°C in water; after 7 days, the test specimens are removed, rinsed with water, blotted, waved back and forth in the air for 15 sec and, 1 min after removal from the water, weighed accurately to 0.1 mg ($m_2$); after this weighing [analogously to 7.12.3.4], the test specimens are dried in a desiccator at 37°C to constant mass, and the mass ($m_3$) is determined accurately to 0.1 mg; water sorption is calculated according to Equation (2) given under 7.12.4.1.

**2.** Milling blank according to Claim 1,

comprising water in an amount of at least 50%, preferably at least 75% and more preferably at least 90% of the water sorption $W_{sp}$
and/or
with modulus of elasticity determined according to ADA Specification No. 27 - 1993 of at least 10 GPa, preferably at least 13 GPa and more preferably at least 15 GPa and/or
with water sorption $W_{sp}$ of at most 40 $\mu$g/mm$^3$, preferably at most 30 $\mu$g/mm$^3$ and more preferably at most 20 $\mu$g/mm$^3$.

**3.** Milling blank according to either of the preceding claims, wherein the quotient of the water sorption $W_{sp}$ and the

modulus of elasticity determined according to ADA Specification No. 27 - 1993 is less than 1.35 $\mu$g/(GPa $\times$ mm$^3$), and preferably less than 1.00 $\mu$g/(GPa $\times$ mm$^3$).

4. Milling blank according to any of the preceding claims, composed of a resin-based composite, comprising

a) an inorganic filler in an amount of at least 70 wt.%, preferably at least 80 wt.%, based on the total mass of the milling blank
and
b) a resin matrix.

5. Milling blank according to Claim 4, wherein the inorganic filler a) comprises:

a1) a glass composition and
a2) non-aggregated and non-agglomerated silica with an average particle size of not more than 80 nm.

6. Milling blank according to Claim 5, wherein the glass composition a1) comprises

a first glass composition a1a) with a D50 in the range of 0.4 to 1.0 $\mu$m, preferably in the range of 0.5 to 0.9 $\mu$m, and
a second glass composition a1b) with a D50 in the range of 1.2 to 5.0 $\mu$m, preferably in the range of 1.5 to 4.0 $\mu$m,
wherein the mass ratio of a1a) to a1b) is between 1:1.5 and 1:8, preferably between 1:2 and 1:5,
wherein the mass ratio of a2) to the total of a1a) and a1b) is between 1:3 and 1:6,
wherein the ratio of the D50 value of the first glass composition a1a) to the D50 value of the second glass composition a1b) is in the range of 1:1.5 to 1:10, preferably 1:2 to 1:5,
and wherein the D75 value of the first glass composition a1a) is less than the D25 value of the second glass composition a1b) .

7. Milling blank according to any of Claims 4 to 6, wherein the resin matrix b) is a polymer of monomers that contain difunctional (meth)acrylates, wherein the percent by weight of ethoxylated bisphenol A dimethacrylate with an average degree of ethoxylation of 2 to 4 ethoxy groups per molecule is greater than 40 wt.% and less than 50 wt.%, based on the total mass of the monomers.

8. Milling blank according to any of the preceding claims, wherein the difference between the water sorption $W_{sp}$ and the water content of the milling blank is less than 10 $\mu$g/mm$^3$, preferably less than 8 $\mu$g/mm$^3$ and preferably less than 4 $\mu$g/mm$^3$.

9. Milling blank according to any of the preceding claims, the dimensions of which are selected such that

- a cube with an edge length of 10 mm, preferably 14 mm,
and/or
- a cuboid having a square base with an edge length of 10 mm, preferably 14 mm, and a height of 20 mm can be milled from it.

10. Milling blank according to any of the preceding claims, wherein the milling blank is sealed or enclosed in a water-tight manner, preferably enclosed by a water-tight container, preferably a water-tight blister, and/or a water-tight lacquer and/or a water-tight wax sheath.

11. Use of a milling blank according to any of the preceding claims for producing a moulded part for use as an indirect dental restoration, preferably a moulded part selected from the group consisting of an inlay, an onlay, a partial crown, a crown, a veneer and a bridge.

12. Method for producing a milling blank according to any of Claims 1 to 10 or a moulded part produced therefrom for use as an indirect dental restoration, comprising the following steps:

(i) production or provision of a milling blank containing water in an amount of less than 25% of the water sorption $W_{SP}$, preferably less than 15% and more preferably less than 10%,
(ii) establishment of the conditions in which the milling blank provided or produced in step (i) sorbs water and maintenance of these conditions until the milling blank contains water in an amount of at least 25% of the water

sorption $W_{sp}$ and preferably water-tight sealing or enclosing of the milling blank produced.

13. Method according to Claim 12 for producing a moulded part for use as an indirect dental restoration, comprising the following additional step:

(iii) milling of the moulded part from the milling blank produced in step (ii), preferably by means of a CAD/CAM milling device, and preferably polishing of the moulded part.

14. Kit for producing indirect dental restorations, comprising

- two or more milling blanks according to any of Claims 1 to 10 of different colours
and
- one or more dental compositions for bonding a moulded part milled from one of the milling blanks to a dental object in the oral cavity, preferably selected from the group consisting of primers, adhesives and bonding cements,
and preferably
- further accessories such as brushes, polishing agents and transfer needles.

**Revendications**

1. Ebauche à fraiser pour la production d'une restauration dentaire indirecte, composée de plastique ou d'un composite à base de plastique, comprenant de l'eau dans une quantité d'au moins 25 % de l'absorption d'eau $W_{SP}$, dans laquelle l'absorption d'eau ($W_{sp}$) est déterminée de manière analogue à EN ISO 4049:2009 [D] [7.12] ; la méthode de détermination est résumée et les divergences par rapport à la procédure selon EN ISO 4049:2009 [D] sont indiquées dans ce qui suit : à partir de blocs composites produits, des échantillons avec une longueur de 14,7 mm, une largeur de 14,7 mm et une épaisseur de 0,5 mm sont produits avec une scie à grande vitesse, ébavurés, rectifiés et polis ; les échantillons sont séchés [de manière analogue à 7.12.3.1] dans le dessicateur à 37 °C jusqu'à la constance de masse et la masse ($m_1$) est déterminée précisément à 0,1 mg, ainsi que [de manière analogue à 7.12.3.2] la longueur, la largeur et l'épaisseur déterminées précisément à 0,01 mm ; le volume V est calculé à partir de cela en millimètres cubes ; ensuite les échantillons [de manière analogue à [7.12.3.3] sont stockés dans l'eau pendant 7 jours à 37 °C ; 7 jours après les échantillons sont retirés, rincés à l'eau, tamponnés, amenés à pivoter en va-et-vient dans l'air pendant 15 secondes et pesés ($m_2$) précisément à 0,1 mg 1 minute après le retrait de l'eau ; après cette pesée les échantillons de manière analogue à [7.12.3.4] sont séchés dans le dessicateur à 37 °C jusqu'à la constance de masse et la masse ($m_3$) est déterminée à 0,1 mg ; l'absorption d'eau est calculée de manière correspondante à l'équation (2) indiquée en 7.12.4.1.

2. Ebauche à fraiser selon la revendication 1,

comprenant de l'eau dans une quantité d'au moins 50 %, de préférence au moins 75 %, de manière particulièrement préférée au moins 90 % de l'absorption d'eau $W_{sp}$
et/ou
avec un module d'élasticité, déterminé selon la spécification ADA n°27 - 1993, d'au moins 10 GPa, de préférence d'au moins 13 GPa et de manière particulièrement préférée d'au moins 15 GPa
et/ou
avec une absorption d'eau $W_{sp}$ de maximum 40 $\mu$g/mm$^3$, de préférence de maximum 30 $\mu$g/mm$^3$ et de manière particulièrement préférée de maximum 20 $\mu$g/mm$^3$.

3. Ebauche à fraiser selon l'une quelconque des revendications précédentes, dans laquelle le quotient de l'absorption d'eau $W_{sp}$ et du module d'élasticité déterminé selon la spécification ADA n°27 - 1993 est inférieur à 1,35 $\mu$g/(GPa $\times$ mm$^3$), de préférence est inférieur à 1,00 $\mu$g/(GPa $\times$ mm$^3$).

4. Ebauche à fraiser selon l'une quelconque des revendications précédentes, composée d'un composite à base de plastique, comprenant

a) une charge anorganique dans une quantité d'au moins 70 % en poids, de préférence d'au moins 80 % en poids, rapportée à la masse totale de l'ébauche à fraiser
et
b) une matrice de résine.

**5.** Ebauche à fraiser selon la revendication 4, dans laquelle la charge anorganique a) comprend :

a1) une composition de verre et
a2) de l'acide silique non agrégé et non aggloméré avec une taille de particules moyenne non supérieure à 80 nm.

**6.** Ebauche à fraiser selon la revendication 5, dans laquelle la composition de verre a1)

comprend une première composition de verre a1a) avec une valeur D50 dans la plage de 0,4 à 1,0 $\mu$m, de préférence dans la plage de 0,5 à 0,9 $\mu$m,
et
une deuxième composition de verre a1b) avec une valeur D50 dans la plage de 1,2 à 5,0 $\mu$m, de préférence dans la plage de 1,5 à 4,0 $\mu$m,
dans laquelle le rapport de masse de a1a) à a1b) est compris entre 1 : 1,5 et 1 : 8, de préférence entre 1 : 2 et 1 : 5,
dans laquelle le rapport de masse de a2) sur la somme de a1a) et a1b) est compris entre 1 : 3 et 1 : 6,
dans laquelle le rapport de la valeur D50 de la première composition de verre a1a) sur la valeur D50 de la deuxième composition de verre a1b) se situe dans la plage de 1 : 1,5 à 1 : 10, de préférence 1 : 2 à 1 : 5,
et dans laquelle la valeur D75 de la première composition de verre a1a) est inférieure à la valeur D25 de la deuxième composition de verre a1b).

**7.** Ebauche à fraiser selon l'une quelconque des revendications 4 à 6, dans laquelle la matrice de résine b) est un polymérisat de monomères, qui contiennent des (méth)acrylates difonctionnels, dans laquelle la part en poids de bisphénol-A-diméthacrylate éthoxylé avec un degré d'éthoxylation moyen de 2 à 4 groupes éthoxy par molécule est supérieure à 40 % en poids et inférieure à 50 % en poids, rapporté à la masse totale des monomères.

**8.** Ebauche à fraiser selon l'une quelconque des revendications précédentes, dans laquelle la différence entre l'absorption d'eau $W_{sp}$ et la teneur en eau de l'ébauche à fraiser est inférieure à 10 $\mu$g/mm$^3$, de préférence inférieure à 8 $\mu$g/mm$^3$, de préférence inférieure à 4 $\mu$g/mm$^3$.

**9.** Ebauche à fraiser selon l'une quelconque des revendications précédentes, dont les dimensions sont sélectionnées de sorte qu'à partir de celle-ci

- un cube avec une longueur d'arête de 10 mm, de préférence 14 mm,
et/ou
- un parallélépipède rectangle avec une surface de base carrée avec une longueur d'arête de 10 mm, de préférence 14 mm, et une hauteur de 20 mm
peut être fraisé.

**10.** Ebauche à fraiser selon l'une quelconque des revendications précédentes, dans laquelle l'ébauche à fraiser est scellée ou entourée de manière étanche à l'eau, de préférence est entourée par un contenant étanche à l'eau, de préférence un blister étanche à l'eau, et/ou un vernis étanche à l'eau et/ou une enveloppe de cire étanche à l'eau.

**11.** Utilisation d'une ébauche à fraiser selon l'une quelconque des revendications précédentes pour la production d'un corps moulé à utiliser comme restauration dentaire indirecte, de préférence d'un corps moulé sélectionné dans le groupe constitué d'un inlay, onlay, d'une couronne partielle, couronne, facette et d'un bridge.

**12.** Procédé pour produire une ébauche à fraiser selon l'une quelconque des revendications 1 à 10 ou un corps moulé fabriqué à partir de celle-ci à utiliser comme restauration dentaire indirecte, avec les étapes suivantes :

(i) la production ou fourniture d'une ébauche à fraiser comprenant de l'eau dans une quantité de moins de 25 % de l'absorption d'eau $W_{SP}$, de préférence de moins de 15 %, de manière particulièrement préférée de moins de 10 %,
(ii) le réglage de conditions, pour lesquelles l'ébauche à fraiser fournie ou produite à l'étape (i) absorbe l'eau et le maintien de ces conditions, jusqu'à ce que l'ébauche à fraiser comprenne l'eau dans une quantité d'au moins 25 % de l'absorption d'eau $W_{SP}$, ainsi que de préférence le scellement ou le fait d'entourer l'ébauche à fraiser générée de manière étanche à l'eau.

**13.** Procédé selon la revendication 12 pour la production d'un corps moulé à utiliser comme restauration dentaire indirecte, comprenant l'étape supplémentaire :

(iii) de fraisage du corps moulé composé de l'ébauche à fraiser générée à l'étape (ii), de préférence au moyen d'un dispositif à fraiser CFAO, ainsi que de préférence le polissage du corps moulé.

14. Kit pour la production de restaurations dentaires indirectes, comprenant

- deux ébauches à fraiser ou plus selon l'une quelconque des revendications 1 à 10, en différentes couleurs ainsi que
- une ou plusieurs compositions dentaires pour la fixation d'un corps moulé fraisé à partir d'une des ébauches à fraiser sur un objet dentaire dans la cavité buccale, de préférence sélectionnée dans le groupe constitué d'apprêts, adhésifs et ciments de fixation,
ainsi que de préférence
- d'autres accessoires tels que des pinceaux, agents de polissage et canules de mélange.

Fig. 1

SECTION B-B

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2014200284 A
- DE 102017103084
- DE 69922413 T2
- EP 1143915 B1
- DE 3941629 A1
- DE 1816823
- DE 2419887
- DE 2406557
- DE 2931926
- DE 3522005
- DE 3522006
- DE 3703120
- DE 102005021332
- DE 102005053775
- DE 102006060983
- DE 69935794
- DE 102007034457
- DE 102006019092 A1
- DE 3941629 C2
- DE 102006019092
- DE 60116142
- DE 3801511 C2
- DE 102006050153 A1
- EP 0184095 B1
- DE 4231579 C2
- EP 0366977 B1
- US 7081485 B2
- DE 3236026 A1
- US 20070027229 A1
- EP 0262629 B1
- EP 0073413 A
- US 7148382 B2
- US 5761169 A
- DE 19708294 A1
- EP 0057474 A
- EP 0047902 A
- EP 0007508 A
- DE 60029481 T2
- EP 0980682 B1
- EP 0948955 B1
- EP 1236459 B1
- EP 0173567 A2
- EP 1720506 A
- DE 3941629
- EP 1839640 A
- DE 1495520
- WO 02092021 A
- WO 02092023 A
- EP 1872767 A
- EP 2070506 A
- EP 1881010 A
- DE 102007050763
- US 6288138 B
- DE 112006001049
- US 7214726 B
- EP 2070935 A
- EP 0059451 A
- US 20140200284 A1

**In der Beschreibung aufgeführte Nicht-Patentliteratur**

- **J.-P. FOUASSIER.** Photoinitiation, Photopolymerization and Photocuring. Hanser Publishers, 1995
- Radiation Curing in Polymer Science and Technology. Elsevier Applied Science, 1993, vol. II
- **ROGGENDORF MJ ; KUNZI B ; EBERT J ; ROGGENDORF HC ; FRANKENBERGER R ; REICH SM.** Seven-year clinical performance of CEREC-2 all-ceramic CAD/CAM restorations placed within deeply destroyed teeth. *Clin Oral Investig,* 2012, vol. 16 (5), 1413-1424
- **FRANKENBERGER R ; PETSCHELT A ; KRÄMER N.** Leucite-reinforced glass ceramic inlays and onlays after six years: clinical behavior. *Oper Dent,* 2000, vol. 25 (6), 459-465
- **FRANKENBERGER R ; TASCHNER M ; GARCIA-GODOY F ; PETSCHELT A ; KRÄMER N.** Leucite-reinforced glass ceramic inlays and onlays after 12 years. *Adhes Dent,* 2008, vol. 10 (5), 393-398
- **OTTO T ; DE NISCO S.** Computer-aided direct ceramic restorations: a 10-year prospective clinical study of Cerec CAD/CAM inlays and onlays. *Int J Prosthodont,* 2002, vol. 15 (2), 122-128
- **POSSELT A ; KERSCHBAUM T.** Longevity of 2328 chairside Cerec inlays and onlays. *Int J Comput Dent,* 2003, vol. 6 (3), 231-248

- **OTTO T ; SCHNEIDER D.** Long-term clinical results of chairside Cerec CAD/CAM inlays and onlays: a case series. *Int J Prosthodont,* 2008, vol. 21 (1), 53-59
- **REISS B.** Clinical results of CEREC inlays in a dental practice over a period of 18 years. *Int J Comput Dent,* 2006, vol. 9 (1), 11-22
- **KASSEM AS ; ATTA O ; EI-MOWAFY O.** Combined effects of thermocycling and loadcycling on microleakage of computer-aided design/computer-assisted manufacture molar crowns. *Int J Prosthodont,* 2011, vol. 24 (4), 376-378
- **KASSEM AS ; ATTA O ; EI-MOWAFY O.** Fatigue resistance and microleakage of CAD/CAM ceramic and composite molar crowns. *J Prosthodont,* 2012, vol. 21 (1), 28-32
- **ATTIA A ; ABDELAZIZ KM ; FREITAG S ; KERN M.** Fracture load of composite resin and feldspathic all-ceramic CAD/CAM crowns. *J Prosthet Dent,* 2006, vol. 95 (2), 117-123
- **RAMIREZ-SEBASTIA A ; BORTOLOTTO T ; ROIG M ; KREJCI I.** Composite vs Ceramic Computer-aided Design/Computer-assisted Manufacturing Crowns in Endodontically Treated Teeth: Analysis of Marginal Adaptation. *Oper Dent,* 2013, vol. 38 (6), 663-673
- **LAUVAHUTANON S ; TAKAHASHI H ; SHIOZAWA M ; IWASAKI N ; ASAKAWA Y ; OKI M ; FINGER WJ.** Arksornnukit M; Mechanical properties of composite resin blocks for CAD/CAM. *Dent Mater J,* 2014, vol. 33 (5), 705-710
- **HEINTZE SD.** Crown pull-off test (crown retention test) to evaluate the bonding effectiveness of luting agents. *Dental Materials,* 2010, vol. 36 (3), 193-206
- **BÄHR N ; KEUL C ; EDELHOFF D ; EICHBERGER M ; ROOS M ; GERNET W ; STAWARCZYK B.** Effect of different adhesives combined with two resin composite cements on shear bond strength to polymeric CAD/CAM materials. *Dent Mater J,* 2013, vol. 32 (3), 492-501
- **GÜNGÖR MB ; NEMLI SK ; BAL BT ; ÜNVER S ; DOGAN A.** Effect of surface treatments on shear bond strength of resin composite bonded to CAD/CAM resin-ceramic hybrid materials. *Adv Prosthodont,* 2016, vol. 8, 259-266
- **ELSAKA SE.** Bond strength of novel CAD/CAM restorative materials to self-adhesive resin cement: the effect of surface treatments. *Adhes Dent,* 2014, vol. 16 (6), 531-540
- **YOSHIDA K ; KAMADA K ; ATSUTA M.** Effects of two silane coupling agents, a bonding agent, and thermal cycling on the bond strength of a CAD/CAM composite material cemented with two resin luting agents. *J Prosthet Dent,* 2001, vol. 85 (2), 184-189
- **KEUL C ; MÜLLER-HAHL M ; EICHBERGER M ; LIEBERMANN A ; ROOS M ; EDELHOFF D ; STAWARCZYK B.** Impact of different adhesives on work of adhesion between CAD/CAM polymers and resin composite cements. *J Dent,* 2014, vol. 42 (9), 1105-1114
- **STAWARCZYK B ; BASLER T ; ENDER A ; ROOS M ; OZCAN M ; HAMMERLE C.** Effect of surface conditioning with airborne-particle abrasion on the tensile strength of polymeric CAD/CAM crowns luted with self-adhesive and conventional resin cements. *J Prosthet Dent,* 2012, vol. 107 (2), 94-101
- **GILBERT S.** Einfluss der Testmethode auf den Verbund zwischen CAD/CAM-Hochleistungspolymer und kunststoffbasierten Befestigungsmaterialien nach unterschiedlichen Vorbehandlungen. Ludwig-Maximilians-Universität, 2014
- **MARTIN A.** Zugverbundfestigkeitsuntersuchungen von Hochleistungskunststoffen nach unterschiedlichen Vorbehandlungsmethoden. Ludwig-Maximilians-Universität, 2015
- **STAWARCZYK B ; STICH N ; EICHBERGER M ; EDELHOFF D ; ROOS M ; GERNET W ; KEUL C.** Long-term tensile bond strength of differently cemented nanocomposite CAD/CAM crowns on dentin abutment. *Dental Materials,* 2014, vol. 30 (3), 334-342
- **BASLER T.** Effect of surface conditioning with air-abrasion on the tensile strength of polymeric CAD/CAM crowns luted with self-adhesive and conventional resin cements. Universität Zürich, 2011
- **FRANKENBERGER R ; HARTMANN VE ; KRECH M ; KRÄMER N ; REICH S ; BRAUN A ; ROGGENDORF M.** Adhäsive Befestigung neuer CAD/CAM-Materialien. *Int J Comput Dent,* 2015, vol. 18 (1), 9-20
- **SHETTY R ; SHENOY K ; DANDEKERI S ; SUHAIM KS ; RAGHER M ; FRANCIS J.** Resin-Matrix Ceramics - An Overview. *International Journal of Recent Scientific Research,* 2015, vol. 6 (11), 7414-7417
- **VANOORBEEK S ; VANDAMME K ; LIJNEN I ; NAERT I.** Computer-aided designed/computer-assisted manufactured composite resin versus ceramic single-tooth restorations: a 3-year clinical study. *Int J Prosthodont.,* 2010, vol. 23 (3), 223-230
- **SCHEPKE U ; MEIJER HJA ; VERMEULEN KM ; RAGHOEBAR GM ; CUNE MS.** Clinical Bonding of Resin Nano Ceramic Restorations to Zirconia Abutments: A Case Series within a Randomized Clinical Trial. *Clin Implant Dent Relat Res,* 2016, vol. 18 (5), 984-992